# EUROPEAN PATENT APPLICATION

(11) **EP 4 775 199 A1**
(43) Date of publication of application: **15.07.2026**
(21) Application number: 23789333.4
(22) Date of filing: 05.09.2023
(51) Int. Cl.: A61K 9/00, A61K 47/42, A61L 31/00, A61N 1/00

(54) **DRUG-RELEASING ELECTROACTIVE IMPLANT FOR NERVOUS SYSTEM REPAIR**

(71) Applicant: Fundación Hospital Nacional de Parapléjicos Para la Investigación y la Integración (FUHNPAIIN), 45071 Toledo (ES)
(72) Inventor: COLLAZOS CASTRO, Jorge Eduardo, 45071 Toledo (ES); ALVES SAMPAIO, Alexandra Manuela, 45071 Toledo (ES)
(74) Representative: Pons IP
(86) International application number: PCT/ES2023/070538
(87) International publication number: WO 2025/052005

(57) **Abstract**

The present invention relates to a filler hydrogel which is implanted in neurological damage sites and releases drugs with antifibrotic and neural growth-promoting activity, and to the combined use thereof with electrical stimulation and with other drugs to facilitate nervous system repair *in vivo*; as well as to compounds, methods, and procedures for preparing and implementing said implants.

## Description

The present invention relates to a volumetric or tissue filler implant which releases drugs with antifibrotic and neural growth-promoting activity and provides electrical stimuli, and to the combined use thereof with electrostimulation protocols and with other drugs to promote nervous system regeneration *in vivo*; as well as to compounds, methods, and procedures for preparing and implementing said implants, and to neural electrostimulation systems which incorporate the compounds or are co-administered with the compounds to prevent fibrosis and induce axonal or dendritic growth on or around the implanted electrodes.

### BACKGROUND OF THE INVENTION

The nervous system (NS) is damaged in a wide variety of pathological processes including trauma, hemorrhage, and vascular events that lead to hypoxia and ischemia, infections, inflammatory disorders, genetic, metabolic, toxic, and neurodegenerative diseases. In clinical practice, these pathologies can affect any region of the central nervous system (CNS), made up of the brain and spinal cord, or involve the peripheral nervous system (PNS), formed by nerves which transport sensory and motor information from/to all organs of the body. Secondary functional loss to neurological injury depends on the location and severity of the injury.

Traumatic spinal cord injury is a common and well-studied type of neurological damage that is, however, still without any effective treatment. The pathophysiology of spinal cord injury (SCI), as well as the strategies proposed for its repair, are described in detail in different publications (Collazos-Castro JE. Spinal cord injury and repair: neuropathological and functional aspects. In: Esclarín de Ruz A. (Ed): Spinal cord injury, multidisciplinary approach. Editorial Médica Panamericana. Madrid, 2020, pages 235-241. Collazos-Castro JE. Biomaterial-based systems as biomimetic agents in the repair of the CNS. In: Salgado A. (Ed): Handbook of Innovations in Central Nervous System Regenerative Medicine. Elsevier, Amsterdam, 2020). In summary, SCI disrupts the axons (axotomy) that run through the affected site, and also causes the death of neurons, glial, vascular, and meningeal cells, and is accompanied by a series of complex responses that lead to tissue scarring without the functional anatomy of the NS being restored.

In humans, traumatic SCI usually causes significant defects in neural tissue, ranging from 1 to 10 cm in length or even more. After the acute phase in which glia and inflammatory cells phagocytize the devitalized tissue, cavities are formed with a variable component of fibrotic connective tissue. Pericytes, perivascular and meningeal fibroblasts, other meningeal cells, and fibroblasts of spinal ligaments contribute to the latter (Fernandez E, Pallini E. Connective tissue scarring in experimental spinal cord lesions: significance of dural continuity and role of epidural tissues. Acta Neurochir. (Vienna) 1985; 76: 145-8; Göritz C, Dias DO, Tomilin N, Barbacid M, Shupliakov O, Frisén J. A pericyte origin of spinal cord scar tissue. Science 2011; 333:238-42; Soderblom C, Luo X, Blumenthal E, Bray E, Lyapichev K, Ramos J, Krishnan V, Lai-Hsu C, Park KK, Tsoulfas P, Lee JK. Perivascular fibroblasts form the fibrotic scar after contusive spinal cord injury. J Neurosci. 2013; 33:13882-7; Alves-Sampaio A, Del-Cerro P, Collazos-Castro JE. Composite Fibrin/Carbon Microfiber Implants for Bridging Spinal Cord Injury: A Translational Approach in Pigs. Int J Mol Sci. 2023;24:11102). Cavities and scars in connective tissue constitute a mechanical and molecular impediment to axonal regeneration and glial migration, and interfere with the presentation of topographical and molecular signals for neural cell growth. For effective treatment of neurological damage, said cavities must be filled with volumetric implants that do not increase fibrosis and provide guidance and stimulus for neural regeneration.

Microtubules are a relevant target to try to increase axonal regeneration. Patent WO 2006/094811 claims the use of microtubule-stabilizing drugs, for example, taxol, for preparing pharmaceutical compounds that stimulate axonal growth after CNS injury. Since correct microtubule dynamics are also indispensable for cell migration and division, microtubule-stabilizing drugs significantly affect these processes, and may thereby reduce fibrosis. Administration of taxol decreased fibrotic tissue formation and caused certain axonal growth in rats with SCI (Hellal F, Hurtado A, Ruschel J, Flynn KC, Laskowski CJ, Umlauf M, Kapitein LC, Strikis D, Lemmon V, Bixby J, Hoogenraad CC, Bradke F. Microtubule stabilization reduces scarring and causes axon regeneration after spinal cord injury. Science 2011;331:928-931). However, there is much doubt among experts regarding the potential usefulness of taxol and related drugs in promoting neurological injury repair due to their neurotoxicity and to the fact that they block the migration and division of glial cells and other repair cells, leading to the formation of empty cavities in the spinal cord rather than promoting tissue gap closure (Popovich PG, Tovar CA, Lemeshow S, Yin Q, Jakeman LB. Independent evaluation of the anatomical and behavioral effects of Taxol in rat models of spinal cord injury. Exp. Neurol. 2014;261: 97-108).

While taxol stabilizes microtubules, there are drugs that have the opposite effect, in other words, that increase microtubule mobility, for example, kinesin inhibitors. These inhibitors, and particularly kinesin Eg5 inhibitors, also increase axonal growth and have been proposed for the treatment of nervous system injuries (Lin S, Liu M, Son YJ, Timothy Himes B, Snow DM, Yu W, Baas PW. Inhibition of Kinesin-5, a microtubule-based motor protein, as a strategy for enhancing regeneration of adult axons. Traffic. 2011; 12:269-86; Baas PW, Matamoros AJ. Inhibition of kinesin-5 improves regeneration of injured axons by a novel microtubule-based mechanism. Neural Regen Res. 2015;10:845-9). A preliminary study evaluated the usefulness of monastrol, a low-affinity Eg5 inhibitor, combined with peripheral nerve grafts and chondroitinase ABC to increase axonal regeneration in rats with SCI, (Xu C, Klaw MC, Lemay MA, Baas PW, Tom VJ Pharmacologically inhibiting kinesin-5 activity with monastrol promotes axonal regeneration following spinal cord injury. Exp Neurol. 2015 Jan;263:172-6). Peripheral nerve graft acted as a substrate and guide for axonal growth across the injury site, and chondroitinase ABC acted as an element which causes the degradation of axonal regeneration-inhibiting proteoglycans. Monastrol, administered in solution for 14 days through an intrathecal tube in the site of the implant, had an additional axonal growth-promoting effect, although with no effect on functional recovery.

Various Eg5 inhibitors with much higher affinity and selectivity than monastrol have been developed (Myers SM, Collins I. Recent findings and future directions for interpolar mitotic kinesin inhibitors in cancer therapy. Future Med Chem. 2016;8:463-89), giving them greater therapeutic potential. There are several patents claiming these compounds and the use thereof as antimitotics for the treatment of cancer, for example, patents US8765817 and US20150352114.

Nevertheless, the usefulness of Eg5 inhibitors for the treatment of fibrosis in the NS or other body organs has not been investigated.

Tyrosine kinase receptor-inhibiting drugs may also have beneficial effects following neurological injury, including a decrease in fibrosis. For example, platelet-derived growth factor receptor (PDGFR) inhibitors, such as imatinib, reduce fibrosis in various animal models of skin, lung, and liver disease (Distler JH, Jün A, Huber LC, Schulze-Horsel U, Zwerina J, Gay RE, Michel BA, Hauser T, Schett G, Gay S, Distler O. Imatinib mesylate reduces production of extracellular matrix and prevents development of experimental dermal fibrosis. Arthritis Rheum. 2007;56:311-22; Rhee CK, Lee SH, Yoon HK, Kim SC, Lee SY, Kwon SS, Kim YK, Kim KH, Kim TJ, Kim JW. Effect of nilotinib on bleomycin-induced acute lung injury and pulmonary fibrosis in mice. Respiration. 2011;82:273-87; Pesce A, Ciurleo R, Bramanti A, Armeli Iapichino EC, Petralia MC, Magro GG, Fagone P, Bramanti P, Nicoletti F, Mangano K. Effects of Combined Admistration of Imatinib and Sorafenib in a Murine Model of Liver Fibrosis. Molecules. 2020;25:4310). These compounds have also been proposed for the treatment of neurological injuries. For example, anti-inflammatory, neuroprotective, and antifibrotic effects have been reported for imatinib in rodents with SCI (Abrams MB, Nilsson I, Lewandowski SA, Kjell J, Codeluppi S, Olson L, Eriksson U. Imatinib enhances functional outcome after spinal cord injury. PLoS One. 2012;7(6):e38760), but independent researchers did not obtain the same results with this medication (Sharp KG, Yee KM, Steward O. A re-assessment of treatment with a tyrosine kinase inhibitor (imatinib) on tissue sparing and functional recovery after spinal cord injury. Exp Neurol. 2014 Apr;254:1-11). Although its usefulness is controversial, clinical trials evaluating the safety of imatinib administration in people with cervical spinal cord injury are being carried out at present (Treatment of Cervical Spinal Cord Injury With Imatinib - a Safety and Feasibility Study. ClinicalTrials.gov Identifier: NCT02363361).

Based on the foregoing it is evident that the control of fibrosis after NS injuries is an unresolved problem that needs to be addressed in order to help neural repair. The complete inhibition of fibrosis or the use of neurotoxic or non-specific cytoskeleton-stabilizing drugs, such as taxol, may cause extension of the tissue defect and complete failure of repair responses. The usefulness of the new Eg5-selective inhibitors for decreasing fibrosis and increasing neural growth is unknown, nor is the manner in which these drugs could be administered and combined to have a positive effect on neural repair obvious to one skilled in the art. Moreover, it is believed that Eg5 inhibitors can negatively affect memory and learning (Freund RK, Gibson ES, Potter H, Dell'Acqua ML. Inhibition of the Motor Protein Eg5/Kinesin-5 in Amyloid β-Mediated Impairment of Hippocampal Long-Term Potentiation and Dendritic Spine Loss. Mol Pharmacol. 2016;89:552-9), so it would be necessary to administer them directly to the injury site in order to limit unwanted effects. Other drugs, such as imatinib, seem to be beneficial in SCI, but there is much doubt about such effects as they were not reproduced when evaluated by independent researchers.

Moreover, another multifunctional tool that can help in the repair of various tissues is electrical stimulation since it has been shown to increase cell migration and division, as well as trophic factor secretion (Rajendran S.B., Challen K, Wright KL, Hardy JG. Electrical Stimulation to Enhance Wound Healing. J Funct Biomater. 2021;12:40).

Although previous attempts have failed to facilitate axonal regeneration across CNS injury by means of electrostimulation, they have shown that it can induce some axonal growth responses (Martin JH. Neuroplasticity of spinal cord injury and repair. Handb Clin Neurol. 2022;184:317-330). For example, in rodents, it increases branching of intact corticospinal tract axons (Brus-Ramer M, Carmel JB, Chakrabarty S, Martin JH. Electrical stimulation of spared corticospinal axons augments connections with ipsilateral spinal motor circuits after injury. J Neurosci. 2007;27:13793-801). Nevertheless, there is evidence that it may also have the opposite effect, in other words, inhibit axonal regeneration (Enes J, Langwieser N, Ruschel J, Carballosa-Gonzalez MM, Klug A, Traut MH, Ylera B, Tahirovic S, Hofmann F, Stein V, Moosmang S, Hentall ID, Bradke F. Electrical activity suppresses axon growth through Ca(v)1.2 channels in adult primary sensory neurons. Curr Biol. 2010;20:1154-64).

Low-frequency (20 Hz) electrical stimuli increased the speed of motor axon regeneration in the PNS (Al-Majed AA, Neumann CM, Brushart TM, Gordon T. Brief electrical stimulation promotes the speed and accuracy of motor axonal regeneration. J Neurosci. 2000;20:2602-8), but not in the spinal cord, even when peripheral nerve fragments were simultaneously implanted in the injured site (Harvey PJ, Grochmal J, Tetzlaff W, Gordon T, Bennett DJ. An investigation into the potential for activity-dependent regeneration of the rubrospinal tract after spinal cord injury. Eur J Neurosci. 2005;22:3025-35).

A strategy being explored for neural repair consists of applying electrical stimuli through electroconductive materials implanted inside the injury site, which simultaneously provide substrate and guidance for neural growth. *In vitro*, glia and neurons grow very well on metallic or carbon electrodes coated with conducting polymers biofunctionalized with molecules that facilitate cell adhesion (Collazos-Castro JE, Hernández-Labrado G, Polo JL, García-Rama C. N-Cadherin and L1-functionalised conducting polymers for synergistic stimulation and guidance of neural cell growth. Biomaterials 2013;34:3603-3617). Electrostimulation through these cell substrates can act directly on neurons or glia or indirectly influence them by means of the modification or electrosecretion of molecules present in the electrode/cell interface (Collazos-Castro JE, Polo JL, Hernández-Labrado G, Padial-Cañete V, García-Rama C. Bioelectrochemical Control of Neural Cell Development on Conducting Polymers. Biomaterials 2010;31:9244-9255; Collazos-Castro JE, García-Rama C, Alves-Sampaio A. Glial progenitor cell migration promotes CNS axon growth on functionalized electroconducting microfibers. Acta Biomater. 2016;35:42-56).

In this sense, previous patents (P201231969, PCT/ES2013/070879) have described electroconducting microfibers modified with molecules that promote axonal growth and glial migration over long distances *in vitro*. From a neurophysiological point of view, microfibers allow ultrasensitive recording of neuronal activity (Vara H, Collazos-Castro JE. Biofunctionalized Conducting Polymer/Carbon Microfiber Electrodes for Ultrasensitive Neural Recordings. ACS Appl Mater Interfaces. 2015;7:27016-26), and also better electrical activation of spinal neural circuits (Vara H, Collazos-Castro JE. Enhanced spinal cord microstimulation using conducting polymer-coated carbon microfibers. Acta Biomater. 2019;90:71-86). These microfibers, incorporated in alginate hydrogels for implantation thereof in rodents with SCI, also partially decreased fibrosis and served as a scaffold for cell growth in cavities formed in the neural tissue, promoting axonal regeneration and directed cell migration (Alves-Sampaio A, García-Rama C, Collazos-Castro JE. Biofunctionalized PEDOT-coated microfibers for the treatment of spinal cord injury. Biomaterials 2016; 89:98-113). Despite these histological effects, the implantation of microfibers in alginate hydrogel also increased the neural tissue gap, such that there was no possibility of functional reconnection of the spinal cord. In an attempt to reduce the additional neural damage associated with microfiber implantation, recently reported studies have replaced alginate with fibrin hydrogel (Escarrat V, Perez-Sanchez J, El-Waly B, Collazos-Castro JE, Debarbieux F. Composite Fibrin and Carbon Microfibre Implant to Modulate Postraumatic Inflammation after Spinal Cord Injury. Cells. 2023 12:839; Alves-Sampaio A, Del-Cerro P, Collazos-Castro JE. Composite Fibrin/Carbon Microfiber Implants for Bridging Spinal Cord Injury: A Translational Approach in Pigs. Int J Mol Sci. 2023;24:11102). In these studies, fibrin bundles with microfibers were introduced into the spinal cord injury site in mice or pigs, and although the implant induced some guided axonal growth, no regeneration of supraspinal axons across the injury or functional recovery was obtained, and furthermore the volume of the injury increased significantly in the porcine model.

Taking into account the described state of the art, it is concluded that functional repair of the central nervous system is a multifaceted problem that remains unsolved. In particular, the need to develop filler implants that decrease fibrosis and simultaneously reduce cavitation and the extension of neural damage, while at the same time stimulating neural growth across the injury site and achieving functional recovery has been detected. These multiple effects are indispensable for successful neural tissue repair, and are achieved in the present invention as a result of the combination of a neural tissue filler implant made up of biomaterials and drugs, combined with electrostimulation. More specifically, the problem is solved by an implant that fills the cavity secondary to neurological injury, releases antifibrotic drugs, and applies electrical stimuli to the damaged site of the nervous system, and which can furthermore be combined with the administration of other compounds locally or systemically to improve the restoration of tissue architecture.

### DESCRIPTION OF THE INVENTION

The present invention is comprised in the technical field of the development of implants for inducing nervous system regeneration, as well as of combinations of biomaterials, drugs, and electrostimulation for neurological repair. The invention provides compounds, methods, and devices for filling neural tissue cavities or defects and controlling fibrosis following neurological damage, as well as for promoting, stimulating, and directing axonal growth and the adhesion, proliferation, migration, and differentiation of glial cells, neurons, vascular cells, connective tissue cells, and other types of cells that may contribute to the repair of neurological injuries.

More specifically, this invention relates to filler implants which release drugs with antifibrotic and neural growth-promoting activity, and to the combined use thereof with electrostimulation protocols and with other drugs to facilitate nervous system repair *in vivo*. This invention also relates to compounds, methods, and procedures for preparing and implementing said implants. The implant comprises a hydrogel which fills the tissue defect and contains drugs for local release, and electrodes for applying electrical stimuli. The electrodes can be incorporated inside the hydrogel, usually in the form of microfibers or microwires, to act as a substrate for directed cell growth, or can be positioned adjacent to the hydrogel to electrically stimulate perilesional structures and facilitate neural growth across the injury.

The combination of drugs, biomaterials, and electrical stimulation in this invention provides synergistic effects in neural regeneration which cannot be obtained through said interventions individually. Effects on tissue repair are obtained through the combined action of the hydrogel which fills the injury and thereby prevents blood and cell debris from building up, and simultaneously promotes tissue growth, the drugs released by the hydrogel, which decrease fibrosis and increase glial migration and axonal extension and branching, and electrical stimulation which increases repair responses.

Furthermore, the implant may contain microfibers or microwires that guide cells and axons across the injury.

Therefore, this invention is industrially applicable in the areas of regenerative medicine, biomedicine, biotechnology, and bioengineering and tissue engineering. More specifically, the invention is applicable in the medical industry to promote neural tissue repair and functional restitution after neurological injuries, and in the electrical neuroprothesis industry to stimulate or record neural electrical activity and replace lost functions. The present invention is also applicable in the biotechnology industry, particularly in the design of devices for inducing the proliferation, migration, and selection of glial cells and neural progenitors and axonal growth *in vitro*. Lastly, the applications can be extended to the engineering of tissues and organs other than the nervous system.

The present invention presents the following advantages that give it great potential for industrial development, between them:
1) It identifies efficient pharmacological compounds, doses, and dosage forms to reduce neurological injury-induced fibrosis. The drugs can be applied locally on the injury sites, formulated in a slow reabsorption filler hydrogel, thereby preventing their systemic toxicity and without the need to implant pumps and probes for the continuous release thereof in the neural tissue.
2) It develops a new implant for injured nervous system, wherein the filler hydrogel which releases the drugs is combined with electrostimulation, inducing a cell growth response that promotes tissue defect closure and that cannot be obtained with the hydrogel, the drugs, or electrical stimulation alone. The electrodes can be incorporated inside the hydrogel to further serve as a support and guide for neural growth, or can be positioned adjacent to the hydrogel to stimulate perilesional cells. The implant does not contain cells, and therefore does not have limitations and complications in cell therapy or tissue grafts.
3) It develops a methodology for applying the implant in neurological injuries such as those occurring in humans. The implant can be readily configured for injuries of different shape and size, and accordingly can be transferred to clinical practice.
4) The implant can be combined with the local or systemic administration of other drugs to improve tissue repair responses.

The tissue filler hydrogel can be manufactured from various compounds approved for clinical use, for example, fibrin, and its main pharmacological component is a compound which reduces cell proliferation without exhibiting any neurotoxic activity in nanomolar concentration, preferably a molecule which inhibits or blocks the function of kinesins, particularly kinesin-5, also referred to as Eg5 or KIF11. As exemplified in the present invention, Eg5 is expressed abundantly in connective tissue cells (fibroblasts, pericytes, meningeal cells) that proliferate and form a fibrotic scar which prevents tissue repair following a spinal cord injury, and the drugs inhibiting same, for example, the compound SB743921, reduce or even eliminate fibrous tissue formation and increase axonal growth in the injury site. In the present invention, Eg5 inhibitor drugs are incorporated in fibrin hydrogels without losing their activity and are released for sufficient periods of time to control fibrosis and promote repair responses of neural tissue. However, without electrical stimulation and elements for cell adhesion and guidance, tissue defect closure and neural growth that occur following the implantation of the hydrogel is incomplete and unorganized, being ineffective for functional repair.

To organize and improve neural growth in the injury site, induce neural activity, and achieve functional tissue repair, the implant object of this invention includes electrodes that can be of two types:
- Internal electrodes, in other words, electrodes located inside the hydrogel, in which case biofunctionalized and aligned electroconductive structures such as, for example, carbon microfibers or nanostructures or metallic microwires coated with biomolecules that facilitate adhesion and directed cell growth, are preferred;
- External electrodes, in other words, electrodes located outside the hydrogel, usually adjacent to the hydrogel or on the layers of connective tissue enveloping the neural tissue, such as the meninges of the central nervous system. These electrodes can have any morphology that adapts to the structures on which they are placed.

Moreover, the complexity of the neurological injuries and of the inflammatory and fibrotic responses that occur with respect to any implant in the nervous system may still require combining the implant object of this invention with the administration of other drugs to obtain the desired reparative effects. As shown in the following examples, the PDGFR inhibitor drugs such as imatinib are very useful to enhance the benefits of the implant on neural tissue repair.

Therefore, a first aspect of the invention relates to a drug-releasing, electroactive, volumetric implant comprising:
a) a tissue filler hydrogel made of natural or synthetic polymers selected from the list comprising: polypeptides, fibrin polymers, fibronectin polymers, collagen, proteoglycans, or glycosaminoglycans, alginate, chitosan, acrylamide, methacrylate, hydrogelatin, and derivatives and combinations of these materials; preferably, a fibrin hydrogel, (the hydrogel is obtained by means of gelling of the mentioned polymers),
b) at least one drug incorporated in the preceding hydrogel to control fibrosis and promote axonal growth and glial migration across the injury site, wherein one of the drugs is a compound which blocks or inhibits the function of kinesins, or reduces the expression thereof, preferably kinesin Eg5 inhibitors, and more preferably the drug SB743921;
c) a set of electrodes for applying electrical stimuli on injured tissue, wherein at least one electrode is positioned inside the hydrogel or adjacent thereto.

Preferably, the set of electrodes comprises at least two electrodes.

A "volumetric" implant, also known as "filler implant", is configured for fill a cavity in the tissue or add volume thereto. Accordingly, the implant of the present invention takes the shape of the site or cavity to be filled.

In a preferred embodiment, at least one electrode is positioned adjacent to the hydrogel, in the damaged sites of the nervous system, in direct contact with neural tissue or on connective tissue sheaths.

In another preferred embodiment, at least one electrode is positioned inside the hydrogel and additionally serves as a support, guide, and stimulus for cell growth in the injury site.

In another preferred embodiment, the electrodes inside the hydrogel comprise or consist of: carbon microfibers, carbon nanostructures, or metallic microwires made of, for example, steel, gold, platinum, platinum/iridium, or other metals and the oxides, composites, and alloys thereof, with composite being understood to mean a heterogeneous mixture of these metals with other synthetic or natural non-metallic components to form a single compound.

The terms "microfiber" and "microwire" refer to elongated objects having a micrometric diameter. Normally, carbon microfibers are filaments with an average diameter between 1 and 100 µm, and metallic microwires are between 1 and 1000 µm (measured, for example, by means of scanning electron microscopy (SEM)).

The term "carbon nanostructure" refers to carbon structures wherein at least one of their dimensions (on average), generally average diameter, is of a nanometric size, in other words, less than 1 µm (measured, for example, by means of scanning electron microscopy (SEM). The term nanostructure includes, for example, nanoparticles, nanofibers, nanotubes, etc.

In another preferred embodiment, the electrodes outside the hydrogel comprise or consist of carbon electrodes, steel electrodes, iridium electrodes, platinum electrodes, platinum/iridium electrodes, gold electrodes, electrodes made of other metals and the oxides, composites, and alloys thereof. These electrodes can have any morphology that adapts to the structures on which they are placed.

In another preferred embodiment, the electrodes are coated with conducting polymers. Preferably, the conducting polymers are selected from the list comprising: poly(3,4-ethylenedioxythiophene) (PEDOT), polypyrrole, and polyaniline.

In another preferred embodiment, the surface of the electrode or of the conducting polymer is biofunctionalized, in other words, at least one molecule with biological activity is bonded thereto. For the biofunctionalization of the electrodes, bridge molecules and methods described in the literature and in other patents, some of which are incorporated herein as reference (WO2014096489), can be used. In a preferred embodiment, the molecule with biological activity used for the functionalization of the electrodes or of the conducting polymer is selected from cell adhesion molecules, molecules of the extracellular matrix, growth factors, axon or dendrite guiding factors, such as netrins, ephrins, semaphorins, protocadherins or Slit or Wnt proteins, morphogens such as Sonic Hedgehog, proteoglycans, glycosaminoglycans, gangliosides, proteins, peptides, or combinations thereof.

In a preferred embodiment, the drug, preferably an Eg5 inhibitor, more preferably SB743921, is at a concentration of 1 µg to 1 mg per ml of hydrogel.

A second aspect of the present invention relates to the method used for manufacturing the implant, as described above, which comprises preparing a drug-releasing filler hydrogel. The mixing of the solutions required for forming the hydrogel (gelling) can be performed both *in vitro*, in other words, in tubes, molds, plates, or other laboratory materials, and *in vivo*, in other words, directly in the tissue damage site, so that it more accurately adapts to the shape and size of the injury.

In a preferred embodiment, a fibrin hydrogel is prepared by means of mixing:
- a first aqueous solution containing human fibrinogen and factor XIII and
- a second aqueous solution containing thrombin and CaCl₂,
wherein at least one of these solutions contains the drug, preferably an Eg5 inhibitor, which is retained after the gelling of the fibrin, and wherein at least one of these solutions optionally contains the set of electrodes. The set of electrodes is optional, given that they may be positioned adjacent to the hydrogel during the application of the implant in the body.

In a preferred embodiment, the mixing is performed in a ratio of first solution:second solution of between 1:1 and 2:1 in volume.

In another preferred embodiment, the first solution contains human fibrinogen at concentrations of between 0.5 and 100 mg/ml and factor XIII between 3 and 300 U/ml.

In another preferred embodiment, the second solution contains thrombin at concentrations of between 3 and 300 U/ml and CaCl₂ at concentrations of between 0.26 and 26 mM in water, more preferably in water with 0.9% (w/v) NaCl.

In another preferred embodiment, the drug, preferably an Eg5 inhibitor, more preferably SB743921, is at a final concentration of 1 microgram to 1 milligram per millimeter of mixture.

In another preferred embodiment, the drug is first encapsulated in particles, vesicles, liposomes, or the like, preferably having a nanometric or micrometric size, for the purpose of increasing its retention or improving its activity, and then incorporated in the precursor solutions of the hydrogel.

In another preferred embodiment, the electrodes or at least one electrode (carbon microfibers, metallic microwires, or other electroconducting elements) are incorporated aligned in the hydrogel to direct neural growth, during the gelling process *in vitro* or *in vivo*.

In a third aspect, the present invention relates to the volumetric implant of the present invention for use thereof in repairing neural tissue or the nervous system.

In a preferred embodiment, for use thereof in repairing neural tissue, the following steps of a surgical method are carried out:
a. cleaning the neurological injury site of cell debris, blood, and fibrotic adhesions;
b. implanting the filler hydrogel with the drug in the neurological injury site cleaned according to step a) during gelling when it occurs *in vivo*, or after gelling when it occurs *in vitro*;
c. positioning at least one electrode adjacent to the hydrogel when no electrodes have been incorporated inside the hydrogel implanted in step b, or when no electrodes have been implanted in the neurological injury site prior to step b;
d. supplying electrical stimuli through the electrodes.

In a preferred embodiment, additionally, the injury site is washed in step a) with a solution containing the same pharmacological compound present in the hydrogel, and the solution is left in contact with the tissue for several minutes to impregnate the perilesional site with the drug prior to the formation of the hydrogel.

In another preferred embodiment, the hydrogel with the pharmacological compound is formed *in vitro* and is then implanted in the neurological injury site cleaned according to step a).

In another preferred embodiment, the hydrogel with the pharmacological compound is formed *in vitro*, inside a second implantable material, and the resulting composite implant is introduced in the damaged region of the nervous system cleaned according to step a).

In a preferred embodiment, the second implantable material is selected from a list of polymers comprising silicone, polyacrylamide, methacrylate, hydrogelatin, collagen, elastin, fibronectin, and derivatives and combinations of these materials.

In a preferred embodiment, the hydrogel is formed with the pharmacological compound *in vivo*, such that gelling takes place directly in the neurological injury site cleaned according to step a).

In another preferred embodiment, the set of electrodes is implanted in the neurological damage site and the hydrogel with the pharmacological compound is then formed *in vivo*, in other words, inside the neural tissue, surrounding the electrodes and filling the tissue defect.

In another preferred embodiment, the hydrogel is formed *in vitro* with the pharmacological compound and the set of electrodes, and is then implanted in the neurological injury site.

In another preferred embodiment, the hydrogel with the pharmacological compound undergoes gelling *in vivo* inside the tissue defect, and the set of electrodes is positioned adjacent to the hydrogel, inside the nervous system or on the membranes enveloping the nerve tissue (meninges, epineurium, perineurium).

In another preferred embodiment, electrical stimuli are applied through the set of electrodes of the implant for the purpose of facilitating or directing tissue repair responses. Said stimuli can be biphasic or monophasic stimuli having a positive or negative polarity and suitable intensity and frequency to induce neural growth responses. Preferably, the intensity of the electric current applied, in absolute value, is comprised between 1 nanoampere and 20 milliamperes and/or the frequency of the electrical stimuli is comprised between 0.00001 Hz and 100000 Hz.

In a last preferred embodiment, the electroactive implant for use thereof in repairing neural tissue is used in combination with other locally or systematically administered pharmacological compounds which promote implant integration and neural repair. The pharmacological compound used in combination is preferably selected from the list comprising: an anti-inflammatory drug, antifibrotic, antibiotic, neuroprotector, or a neural growth promoter. More preferably, the pharmacological compound used in combination is a tyrosine kinase receptor inhibitor selected from imatinib, sunitinib, sorafenib, masitinib, dasatinib, nilotinib, gefitinib, erlotinib, lapatinib, and tofacitinib. More preferably, the pharmacological compound used in combination is imatinib.

Throughout the description and the claims, the word "comprises" and its variants do not intend to exclude other technical features, additives, components or steps. For those skilled in the art, other objects, advantages and features of the invention may be partially deduced from both the description and the embodiment of the invention. The following examples and figures are provided by way of illustration and are not intended to limit the present invention.

In the same manner, numerous documents are cited throughout this specification of the invention and incorporated herein by reference.

### DESCRIPTION OF THE FIGURES

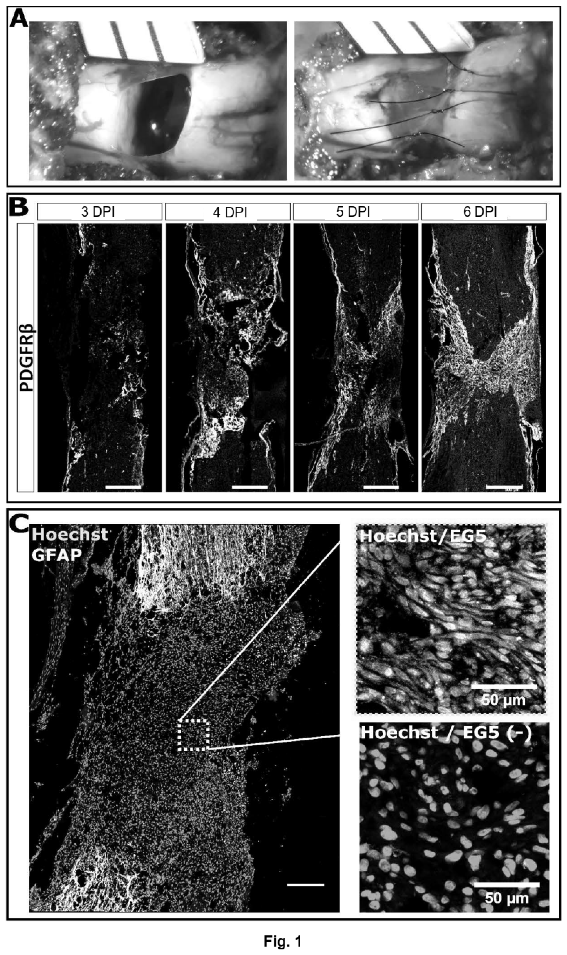

**Figure 1** illustrates the development of fibrotic connective tissue after spinal cord transection in rats.
   **A)** Dorsal view of the surgical procedure carried out on rat spinal cord with a 2 mm excision of neural tissue and subsequent suturing of the dura mater. **B)** Development of PDGFR-positive fibrotic cell proliferation and migration in the injury site. By 6 days post-injury (DPI), a dense fibrotic scar has formed, blocking any possible reconnection of the nervous system. **C)** Most scar cells which intervene in axonal growth and astrocyte (GFAP) migration also express kinesin EG5. A negative staining control for EG5, omitting the primary antibody, is shown in the lower right panel. Tissue sections are 10 µm thick. Scale bars: B, 500 µm; C, left, 200 µm; C right, 50 µm.

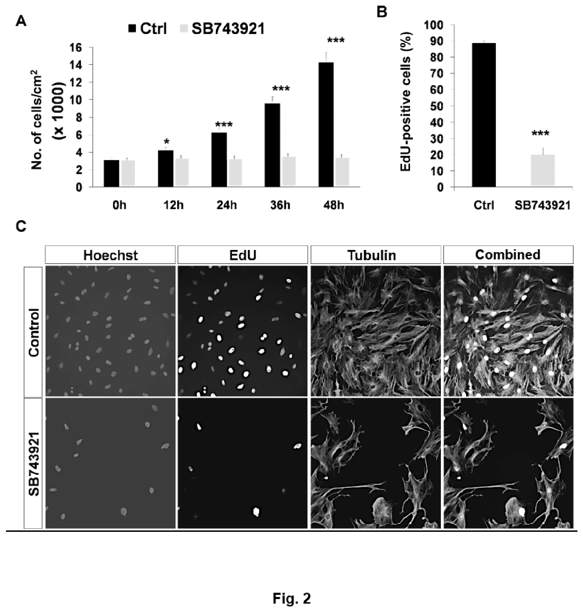

**Figure 2** demonstrates reduction in meningeal fibroblast proliferation after exposure to the compound SB743921, a kinesin EG5 inhibitor.
   **A)** Increase in total cell number over 48 hours in untreated or SB743921-treated cultures. Without treatment, the initial cell number multiplied by 4-5 folds, whereas with treatment the cell number remained constant. **B)** Quantification of cells that incorporated EdU, a DNA synthesis indicator for mitosis. Without the drug, 90% of the cells went into mitosis over the 48 hours of monitoring; whereas only 20% did so in the presence of the drug. **C)** Appearance of untreated (top row) and SB743921-treated (bottom row) cells processed for immunocytochemistry at 48 hours of culture. Treated cells were still alive, but showed alterations in the cytoskeleton (tubulin).

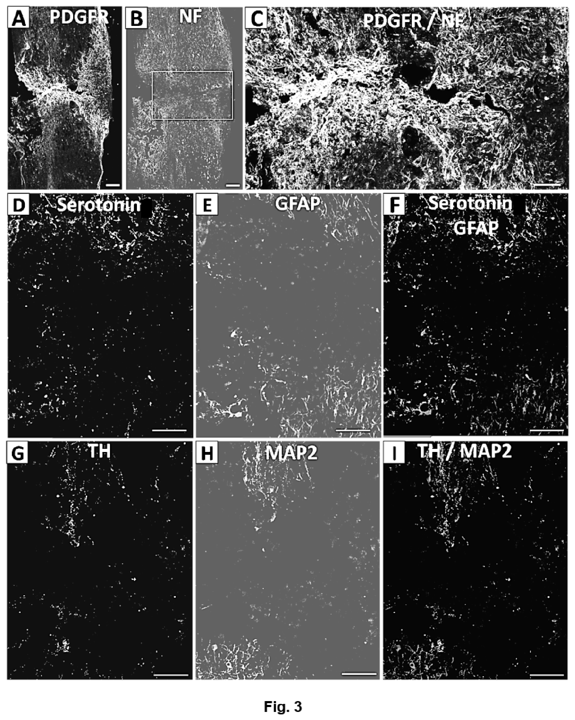

**Figure 3** exemplifies the reduction of fibrosis after spinal cord injury and systemic (subcutaneous) administration of SB743921 in rats.
   **A, B, C)**, Appearance of the injury site at 7 DPI in 10 µm thick histological sections processed for the indicated markers. A partial reduction of fibrosis (PDGFR) was observed after SB743921 administration, which was also more lax and allowed the growth of various neurofilament (NF)-positive axons. Consequently, the stumps moved closer together and the neural tissue gap was reduced to less than 500 µm, as detected by GFAP (**E** and **F**) and MAP2 (**H** and **I**) labeling. Serotonergic (**D** and **F**) and noradrenergic (TH, **G** and **I**) axons entered the scar area, approaching the caudal stump. Scale bars: A, B, 200 µm; C - I, 100 µm.

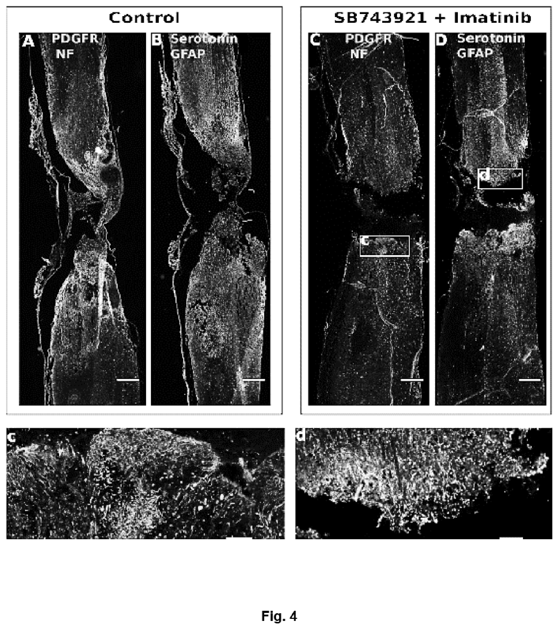

**Figure 4** exemplifies the reduction of fibrosis after spinal cord injury and subcutaneous administration of a combined treatment with SB743921 and imatinib in rats.
   **A, B)** Appearance of the spinal cord in an untreated control animal. A dense PDGFR-positive scar separates the rostral and caudal stumps, creating a neural gap about 2 mm long. Neither axons, (NF, serotonin) nor astrocytes (GFAP) extended into the scar. **C, D)** Appearance of the spinal cord in an animal treated with SB743921 and imatinib. The combination of drugs completely eliminated fibrosis (**C**) partially approaching the stumps of the spinal cord, but without completing tissue defect closure. The regions demarcated by rectangles in **C** and **D** are magnified in the lower part (**c, d**), to illustrate the abundant axonal branching in both the caudal stump (c) and the rostral stump (d). In the absence of a substrate for their growth across the tissue gap, axons accumulated at the edge of the stumps. Scale bars: A-D, 500 µm; b and d, 100 µm.

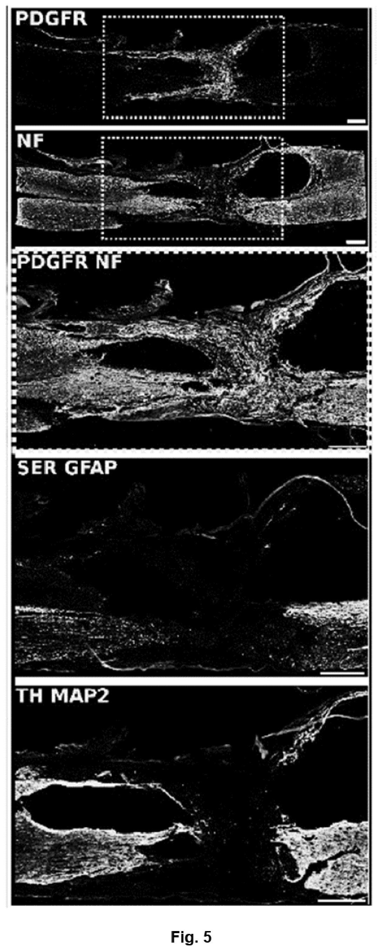

**Figure 5** illustrates tissue cavitation and fibrosis in rats with spinal cord transection and electrostimulation at the injury site, without filler hydrogel implantation or drug administration.
   Biphasic electrical pulses were applied for 10 days to the injury site, implanting the electrodes and using the stimulation protocol with the methods described in detail in Example 5. Spinal cords were extirpated at 14 DPI, cut to 10 µm thick and processed for the markers indicated in each panel of the figure. Electrostimulation did not significantly modify fibrosis (PDGFR) that sealed the stumps, and furthermore increased cavity formation in the spinal cord. Neural cells (astrocytes, GFAP), neurons (MAP2) were separated by the cavities and fibrotic tissue, which also did not allow axons (serotonergic, SER; noradrenergic, TH) to go from one side to the other. Scale bars: 500 µm.

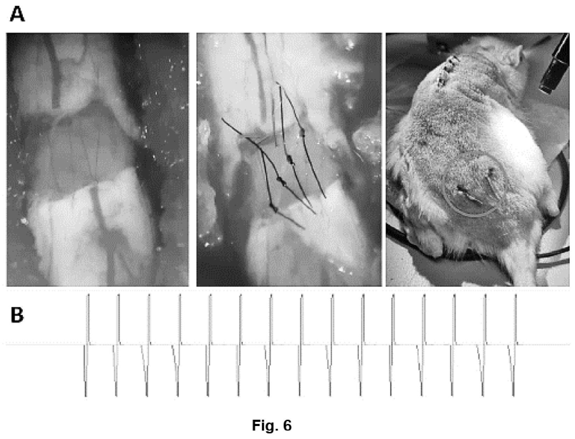

**Figure** 6 exemplifies the implantation of a filler hydrogel formed *in vitro* with fibrin, SB743921, electroconducting microfibers, and the positioning of a microwire adjacent to the hydrogel for electrical stimulation at the spinal cord transection site in rats.
   **A)** The hydrogel implant consisting of fibrin/SB743921 with microfibers was introduced into the 2 mm tissue gap; a stainless steel microwire (50,8 µm in diameter, with Teflon insulation removed from the tip, leaving 1 mm exposed) was then positioned rostrally and dorsally with respect to the implant. Finally, the dura mater was sutured with a 10-0 thread. The counter electrode was placed ventrally, in the subcutaneous abdominal tissue. The wires of the stimulation electrode and the counter electrode ended in gold pins that emerged from the dorso-caudal skin of the animal. **B)** a train of 15 biphasic electrical pulses, with a total duration of 45 ms, was applied every 2 s for 90 minutes for 10 days, starting on the fourth day post-injury.

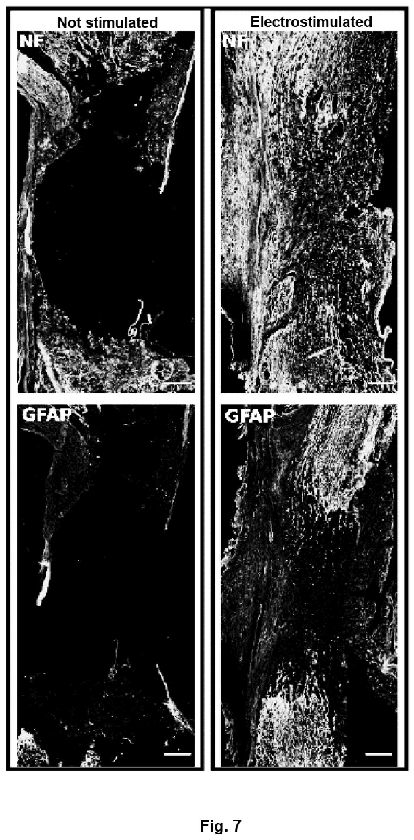

**Figure 7** illustrates the reduction of cavitation, facilitation of neural growth, and closure of spinal cord gap in rats after implantation of the hydrogel formed *in vitro* with fibrin/SB743921/microfibers, combined with electrostimulation.
   At 14 DPI, spinal cords were extirpated and cut into 10 µm thick sections and processed with immunohistochemistry for the specified markers. The state of the spinal cord in animals that received implant with drug, but not electrostimulation, in which an cavity empty of cells was produced in the area of the injury, is illustrated on the **left**. Tissue response to implantation combined with electrostimulation, which closed the tissue defect and caused the growth of various axons (NF) in the injury site, accompanied by migration of astrocytes (GFAP) from the edges of the injury, is shown on the **right**. Scale bar: 250 µm.

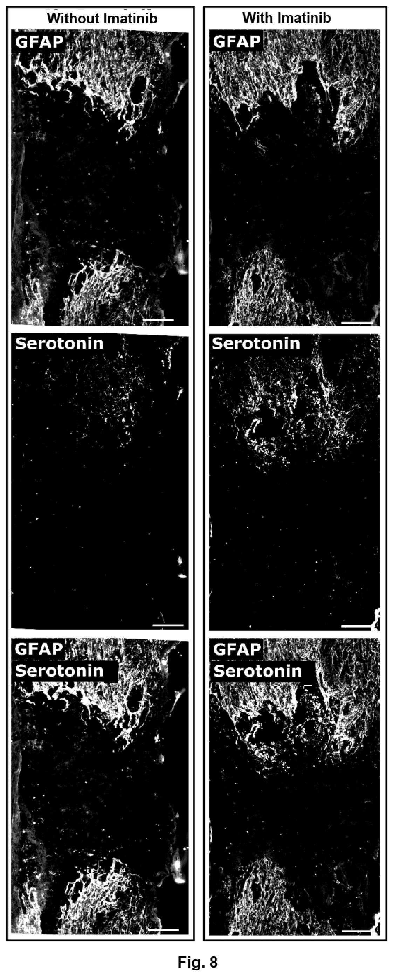

**Figure 8** exemplifies the therapeutic synergy between the SB743921-releasing electroactive implant and the systemic administration of imatinib in rats with spinal cord transection.
   Histological sections of the center of the injury, processed for GFAP and serotonin immunohistochemistry at 14 DPI, are shown. All animals received the implant and electrical stimulation as illustrated in Figures 6 and 7, substantially reducing the neural tissue gap from 2 mm to about 500 µm. It is shown on the **right** that, in animals additionally treated with imatinib, increased axonal branching and growth occurred, in comparison to animals for which the drug was not administered (**left**). Axons exceeded the astrocyte growth front (GFAP), penetrating the injury. The growth of noradrenergic axons and generally neurofilament-labeled axons (not shown) also increased in response to imatinib. Scale bar: 200 µm.

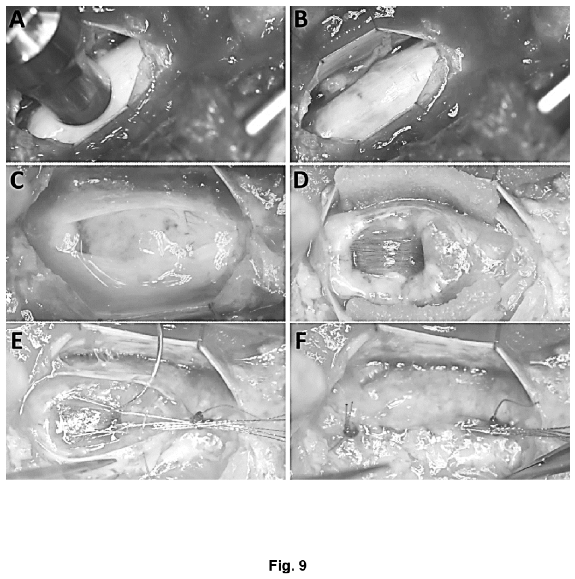

**Figure 9** exemplifies the application of a fibrin hydrogel with SB743921 and carbon microfibers, and the positioning of a set of electrodes adjacent to the hydrogel, for the treatment of neural damage caused by spinal cord contusion in pigs. There are shown intraoperative photographs corresponding to: **A)** moment when the contusion-causing element comes into contact with the porcine spinal cord; **B)** appearance of the traumatized area 24 hours after contusion; **C)** longitudinal incision in the meninges, including the pia, after which a part of the devitalized neural tissue is spontaneously evacuated; **D)** appearance of the area after washing with saline solution and subsequently with a 5 µM solution of SB743921, and introducing a fibrin hydrogel preformed with SB743921 and conducting polymer-coated carbon microfibers; **E)** filling of the rest of the cavity with the same hydrogel gelled *in vivo*, and positioning of electrodes (stainless steel microwires) on the hydrogel. **F)** Suturing of the dura mater.

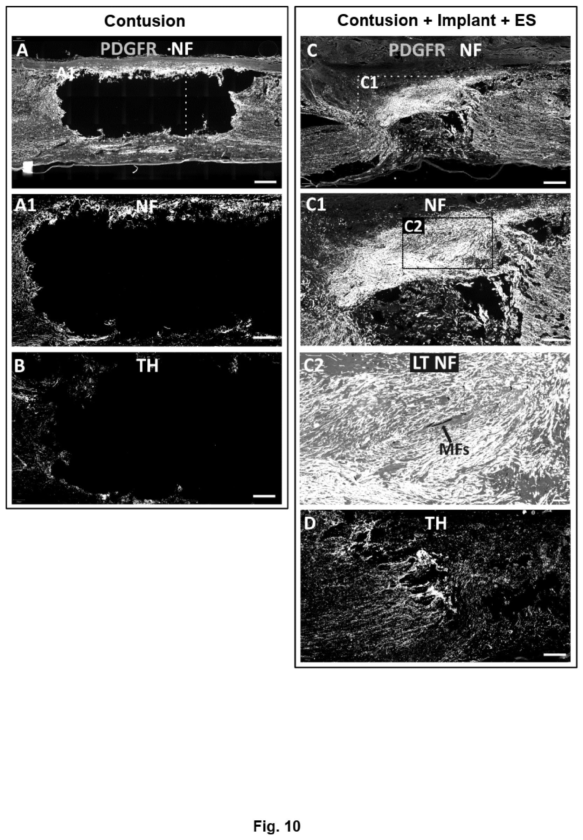

**Figure 10** shows the neural repair obtained by means of implanting a fibrin hydrogel with SB743921 and microfibers, with or without electrical stimulation through metallic microwires positioned on the hydrogel, after spinal cord contusion in pigs. Treatment was applied following the surgical procedure described in Figure 9. For electrostimulation (ES), the same protocol used in rats, described in Example 7, was used, always starting each biphasic pulse with the negative (cathodic) phase. After completing the monitoring, the animals were sacrificed, their spinal cords were extirpated, sectioned to 10 µm thick, and processed for immunohistochemistry of PDGFR, NF, and noradrenergic axons (TH). **A)** Histology of the spinal cord in control animals which suffered contusion without treatment, showing the damaged site ending in a practically empty cavity, with only some remnants of fragmented axons. Furthermore, the spinal tissue showed atrophy on both sides of the injury. The area in the box is shown magnified in (**A1**). **B)** The same area in A1 in a contiguous section stained for TH. Axons end at the rostral edge of the cavity (left), without penetrating it. **C)** Appearance of the spinal cord in animals with contusion plus implantation accompanied by electrical stimulation (ES). The atrophy of the spinal cord has decreased and a bundle of neural tissue with hundreds of axons that have regenerated in the site of the microfiber implant is observed, going through the injury from one side to the other. The axonal bundle is shown magnified in (**C1**), and the box outlined in C1 is in turn amplified in (**C2**), combining fluorescence images with transmitted light (TL) to show the microfibers. **d)** Mass regeneration of noradrenergic axons in the injury site in animals that received the combined treatment. Scale bars: A, and C, 1 mm; A1, C1, B and D, 500 µm; C2, 100 µm.

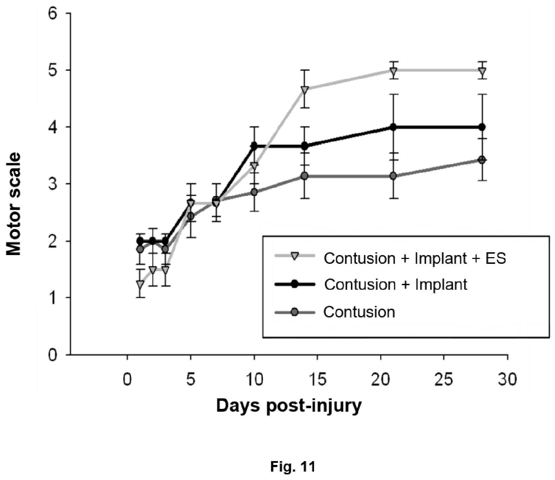

**Figure 11** shows the motor function recovery obtained by means of implanting a fibrin hydrogel with SB743921 and microfibers, with or without electrical stimulation through metallic microwires positioned on the hydrogel, after spinal cord contusion in pigs. Treatment was applied following the surgical procedure described in Figure 9. The functional recovery of the pigs was assessed using the PTIBS scale (score 1, paralysis; score 10, normal locomotion), in the different treatment groups. The implant combined with ES significantly improved (p<0.05 on days 14, 21, and 28 post-injury) the motor functions in pigs with spinal cord contusion.

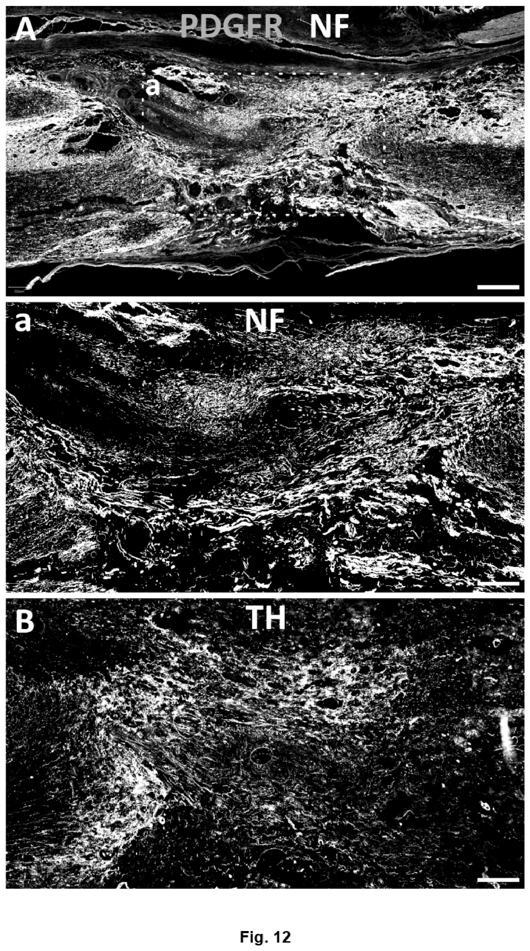

**Figure 12** illustrates the neural repair obtained by means of implanting a fibrin hydrogel with SB743921 and microfibers, combined with electrical stimulation (ES), after spinal cord contusion in pigs. In this case, treatment was applied in the same way as described in Figures 9 and 10, with the difference that the ES pulses always started in the positive (anodic) phase. **A)** This polarity of ES also induced abundant axonal growth across the injury site, evidenced by the presence of NF-positive axon bundles, shown at higher magnification in (**a**). **b**) Noradrenergic axons also grew copiously from one side of the injury to the other. Scale bars: A, 1 mm; a1, and B, 500 µm.

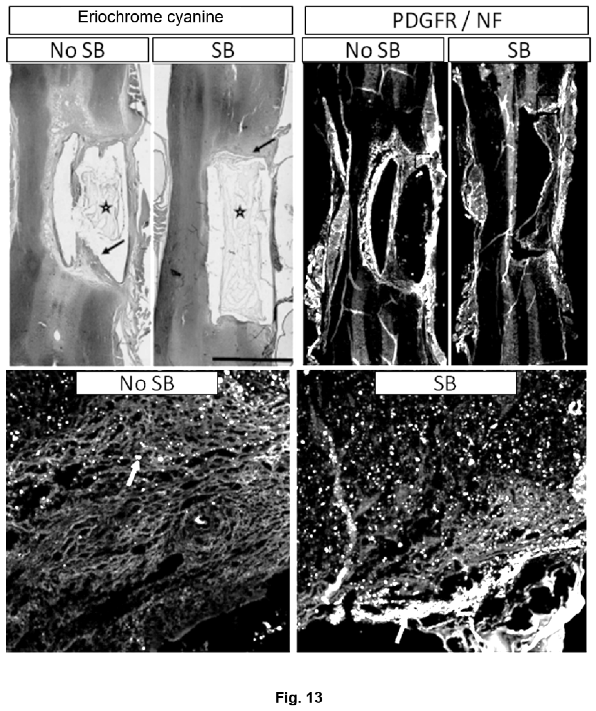

**Figure 13** shows the antifibrotic and neural growth-promoting effect of a hydrogel consisting of fibrin/SB743921 formed *in vitro* and implanted with an additional support material in a cervical spinal hemisection with a 1 cm gap in the neural tissue in pigs.
   A fibrin hydrogel formed *in vitro* with SB743921 (**SB**) or without SB (**No SB**) was implanted inside a silicone tube to provide mechanical support at the injury site. Spinal cords were excised at 30 days post-injury to evaluate cellular responses, and sectioned to 50 µm thick for eriochrome cyanine staining (top left), or to 10 µm thick for PDGFR and NF immunohistochemical staining (top right and magnifications, bottom). The hydrogel degraded only partially in both cases, with remnants remaining contained in the silicone tube (stars). Extensive fibrosis developed in and around the tube in the absence of SB (arrow), but fibrosis was almost completely suppressed when the implants contained SB (arrow). Without the drug, fibrosis was impenetrable to axons, with axonal degenerative bulbs found right at the interface with fibrotic tissue (arrow in lower left panel); whereas, with the drug, various axons were observed growing to the edge of the injury, even when there was some remnant fibrosis (arrow in lower right panel). Scale bar, 5 mm.

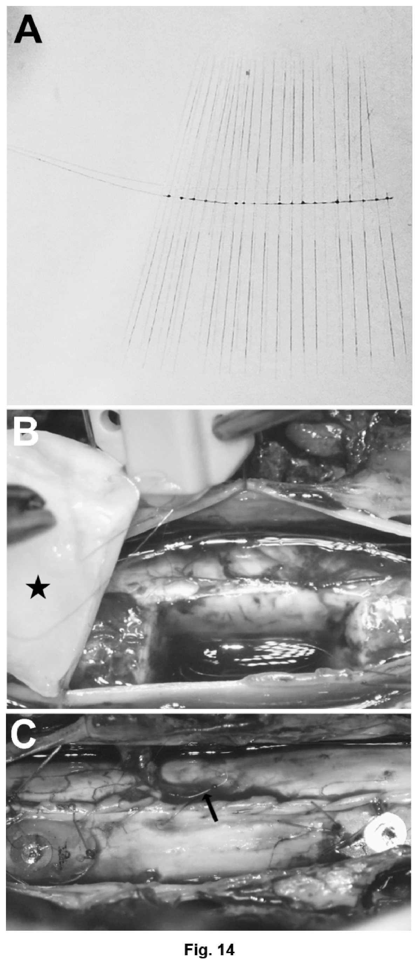

**Figure 14** illustrates the implantation of a fibrin hydrogel formed in vitro with SB743921 and an assembly of interconnected electroconducting microfibers, and epidural electrodes, in a spinal hemisection with tissue excision in pigs.
   **A)** Assembly of carbon microfibers (20 MFs of 12 mm in length), interconnected by a 35N LT microwire of 12.8 µm in diameter and coated with the conducting polymer PEDOT:PSS-*co*-MA. **B)** Implantation of the fibrin hydrogel (star) containing the assembly of electrically interconnected microfibers in the porcine spinal cord injury. **C)** Suturing of the dura mater and epidural fixation of auxiliary metallic electrodes (counter electrode and reference electrode) 5 mm from the edges of the injury. The arrow points to the 35N LT microwire which ends in the connector.

### EXAMPLES

Next, the invention will be illustrated by means of assays carried out by the inventors that demonstrate the effectiveness of the product of the invention.

### Example 1: Development of fibrotic connective tissue after spinal cord transection in rats.

A spinal transection model in adult male Wistar rats was used. Experimental protocols followed European Commission regulations and Spanish legislation for the protection of experimental animals (86/609/EEC, 32/2007, and 223/1988) and were approved by the Ethical Committee for Animal Experimentation of the Hospital Nacional de Parapléjicos of Toledo, Spain. In summary, animals were anesthetized by intraperitoneal (IP) injection of pentobarbital (50 mg/kg) and xylazine (10 mg/kg), providing supplemental doses of 30% one and a half hours later as needed. There were also administered atropine (0.04 mg/kg, IP) to decrease bronchial secretions, marbofloxacin as a prophylactic antibiotic, and ophthalmic ointment to prevent corneal abrasion. A dorsal laminectomy of the T9-T10 vertebrae, under which the T10 and T11 spinal segments are located, respectively, was performed. A small diagonal cut was made in the dura mater, followed by complete spinal cord transection. After a second more rostral cut, the tissue in between was extirpated leaving a neural tissue gap of about 2 mm (Figure 1A). Bleeding was controlled by introducing a fragment of Espongostan^{®} into the cavity, which was removed once hemostasis was achieved. Durorrhaphy was then performed with simple stitches using 10-0 thread; and finally, the muscles and skin were sutured in layers.

Eighteen animals distributed into 6 survival groups from 2 to 7 days after injury were used. The animals were anesthetized and perfused transcardially with saline and 4% paraformaldehyde. Spinal cords were extirpated and immersed in 30% sucrose for 3 days, and then cut into 10-micron sections for histological and immunohistochemical staining. The development of fibrotic connective tissue was assessed by means of immunohistochemical staining for platelet-derived growth factor receptor (PDGFR), which is expressed by pericytes and fibroblasts. The monoclonal antibody used (Abcam [Y92] - C-terminal ab32570) was made in rabbit against a synthetic peptide which is within the human PDGFR, between amino acid 1050 and the carboxyl terminus (Database link: P09619). Furthermore, the expression of kinesin Eg5 in fibrotic tissue (polyclonal antibody GTX30692), and the presence of astrocytes, identified by their expression of glial fibrillary acidic protein (GFAP, BD Biosciences 556327 antibody), were evaluated.

For immunohistochemical staining, tissue sections were blocked for 1 hour at room temperature in phosphate buffered saline (PBS) containing 0.3% triton and 2% goat serum, and incubated, after washing, with primary antibodies overnight at 4°C. The next day, they were treated for 2 hours with fluorescent secondary antibodies (labeled with Alexa-488 or Alexa-594), dissolved in PBS. In all cases, control sections without primary antibodies were included to assess the reliability of staining. Cell nuclei were stained with bisbenzimide (Hoechst 33342, Molecular Probes, 1.5 mg/ml in PBS).

Figure 1B illustrates the proliferation and migration of fibrotic cells from the meninges and perivascular space (fibroblasts and pericytes), which were stained very efficiently by means of immunohistochemistry for PDGFR at the injury site. Invasion of the spinal cavity by said scar cells began on day 2 and was complete by day 6 post-injury, creating a dense fibrotic tissue. In exceptional cases, some scar contraction took place between day 4 and day 6 post-injury, causing the partial approach of the rostral and caudal stumps of the spinal cord at a distance of less than 1 mm (Figure 1B). However, in most cases the fibrotic scar extended 1 to 3 mm in the longitudinal direction of the spinal cord. Axons were always arrested or diverted at the edge of the injury, being unable to go through the scar. Astrocytes also remained confined to the edge of the injury (Figure 1C). The vast majority of fibrotic tissue cells expressed Eg5 (Figure 1C).

### Example 2: Reduction in meningeal fibroblast proliferation after exposure to the compound SB743921, a kinesin Eg5 inhibitor.

After confirming that Eg5 is expressed in fibrotic connective scar tissue which is formed in the spinal cord and generated in much of the meninges, the effect of the compound SB743921 on meningeal fibroblast proliferation was investigated *in vitro*. This compound is a very potent inhibitor (Ki = 0.1 nM) of Eg5 (Myers SM, Collins I. Recent findings and future directions for interpolar mitotic kinesin inhibitors in cancer therapy. Future Med Chem. 2016;8:463-89).

Meningeal fibroblasts were cultured from adult rat dura mater and arachnoid mater in the presence of fetal bovine serum following published procedures (Collazos-Castro JE, Polo JL, Hernández-Labrado G, Padial-Cañete V, García-Rama C. Bioelectrochemical Control of Neural Cell Development on Conducting Polymers. Biomaterials 2010;31:9244-9255). Twenty-four hours after seeding the cells, SB743921 was added at a final concentration of 10 nanomolar (nM). The behavior of living cells was studied by means of videomicroscopy, capturing images every 30 minutes for 48 hours. In separate cultures, cell proliferation was also studied by means of the incorporation of 5-ethynyl-2'-deoxyuridine (EdU). Cells were cultured with or without SB743921 at a concentration of 10 nM, and fixed with paraformaldehyde at 48 hours, after which they were processed to fluorescently reveal EdU, and visualize cells and their nuclei using immunohistochemistry for tubulin and Hoechst, respectively.

Videomicroscopy studies revealed that the division of SB743921-treated meningeal fibroblasts was arrested, with the fibroblasts spending a prolonged period of time in a spherical shape without completing mitosis, and subsequently acquired a heterogeneous aberrant morphology characterized by an increase in cell size, accompanied by enlargement and deformation of the nucleus, as well as flattening of their cytoplasm. However, no direct cytotoxic effect of the drug was observed. SB743921-treated cells lived for 48 hours but maintained their original number, whereas untreated cells multiplied between 4 and 5 folds (P <0.001; Figure 2A). Furthermore, at 48 hours, almost 90 % of the control cells had incorporated EdU, in other words, they had synthesized DNA to divide, in comparison to 20% of cells treated with the drug (P < 0.001; Figures 2B and C).

### Example 3: Reduction of fibrosis after spinal cord injury and systemic administration of SB743921 in rats.

Having demonstrated that SB743921 very efficiently inhibits the proliferation of meningeal fibroblasts *in vitro*, its toxicity *in vivo* and its effect on fibrosis after spinal cord injury in rats were then investigated. The same spinal cord transection model described in Example 1 was used, in other words, a 2 mm neural gap was created and the dura mater was sutured without other treatments at the injured site. The drug was dissolved in water at 0.8 mg/ml and administered subcutaneously (SC).

A total of 30 animals distributed in 5 groups with different treatment regimens with SB743921 were used for this test:
a) 1 mg/kg subcutaneous, one dose every 24 hours for 2, 4, or 6 days, starting 12 hours post-injury.
b) 1 mg/kg, 5 doses in total, every 12 hours, starting 12 hours post-injury.
c) 1 mg/kg, 4 doses in total, every 12 hours, starting 24 hours post-injury.
d) 1 mg/kg, 4 doses in total, every 12 hours, starting 12 hours post-injury.
e) 1,125 mg/kg, 4 doses in total, every 12 hours, starting 12 hours post-injury.

Spinal cord was extirpated at 7 DPI for histological study, since by that time fibrotic connective tissue had sealed the stumps in animals which did not receive any treatment (Example 1 and Figure 1).

Neural damage, Fibrosis development, inflammation, and repair responses (axonal growth, glial migration, blood vessel growth) were evaluated by means of immunohistochemical staining for axons (neurofilament, NF, Sigma-Aldrich N0142; serotonin, ImmunoSolution IG1112; tyrosine hydroxylase, TH, Sigma AB152), neuronal somas and their dendrites (microtubule-associated protein, MAP2, Sigma M1406), astroglia (glial fibrillary acidic protein, GFAP, BD Biosciences 556327); pericytes and fibroblasts (PDGFR, Abcam AB32570; type IV collagen, Meridian, T40251R), macrophages and microglia (ED1; Chemicon MAB 1435; isolectin B4, Fisher I21411), and endothelial cells (RECA1, Bio-Rad, MCA970R). The results obtained are summarized in Table 1.

**Table 1. Effect of the systemic administration of SB743921 in rats with spinal cord transection**

| **Drug regimen** | **Systemic adverse effects** | **Reduction of fibrosis** | **Neural tissue damage** |
|---|---|---|---|
| **a** | No detected in the period of survival (7 days post-injury, DPI). | Slight reduction from the 4th dose. | Severe spinal atrophy with 6 doses. |
| **b** | Mortality >50% at 7 DPI. Diarrhea, peritonitis. | Partial. | Mild microhemorrhages. |
| **c** | Diarrhea, peritonitis, mucositis. | Inconsistent. | Increases due to inflammation. |
| **d** | Diarrhe. Mucositis. | Inconsistent. | Increases due to inflammation. |
| **e** | Mortality >90% at 7 DPI. Diarrhea, peritonitis. | Inconsistent. | Inconsistent. |

As indicated in Table 1, the most effective treatment scheme was "b", which partially reduced fibrosis. Generally, a 300- to 500-micron scar remnant formed in the rostrocaudal direction of the spinal cord, substantially approaching the stumps and accompanied by axonal growth and branching (Figure 3). Axons labeled for neurofilament, serotonin, or tyrosine hydroxylase entered the fibrotic tissue and virtually reached the caudal stump by 7 DPI. There were no obvious signs of neurotoxicity in the perilesional tissue, although tissue damage was detected due to microhemorrhages, with vascular alterations due to inhibition of cell proliferation. Four of the seven animals that received regimen "b" had died 7 DPI with signs of enterocolitis and bacterial translocation or intestinal perforation, and the survivors already showed signs of intestinal involvement.

In conclusion, SB743921 was a partially effective drug in reducing fibrosis and increasing axonal growth when administered systemically, but at effective doses it presented a very high mortality in rats with spinal cord transection, making it necessary to search for therapeutic schemes that are safer and at the same time effective for this purpose.

### Example 4: Reduction of fibrosis after spinal cord injury and subcutaneous administration of a combined treatment with SB743921 and imatinib in rats.

Due to the high toxicity of systemically administered SB743921, the possibility of reducing fibrosis by administering a combination of SB743921 and imatinib systemically was investigated. Imatinib is a PDGFR inhibitor. This receptor is involved in the migration and proliferation of fibroblasts and pericytes during healing of other organs (Rajkumar VS. Platelet-derived growth factor β-receptor activation is essential for fibroblast and pericyte recruitment during cutaneous wound healing. Am J Pathol 2006; 169:2254-65), and as shown in Example 1, it is abundantly expressed in the fibrotic tissue formed after spinal cord injury.

The doses of both drugs were progressively reduced to limit their adverse effects while at the same time finding out the maximum tolerated dose and the effectiveness thereof. The same spinal cord transection model and histological assessment methods described in Examples 1 and 3 were used. Both drugs were administered by subcutaneous injection every 12 hours, starting 12 hours post-injury. A total of 15 rats distributed into 5 groups with the following pharmacological regimens were used for this test:
a) 4 doses of SB743921, 0.8 mg/kg, plus 5 doses of imatinib, 25 mg/kg.
b) 4 doses of SB743921, 0.5 mg/kg, plus 5 doses of imatinib, 25 mg/kg.
c) 4 doses of SB743921, 0.8 mg/kg, plus 5 doses of imatinib, 12.5 mg/kg.
d) 4 doses of SB743921, 0.8 mg/kg, plus 5 doses of imatinib, 6.25 mg/kg.
e) 4 doses of SB743921, 0.65 mg/kg, plus 4 doses of imatinib, 5 mg/kg.

The results obtained are summarized in Table 2.

**Table 2. Effect of the combined subcutaneous administration of SB743921 and imatinib in rats with spinal cord transection**

| **Drug regimen** | **Adverse effects** | **Reduction of fibrosis** | **Neural tissue damage** |
|---|---|---|---|
| **a** | Death 6 to 9 days post-injury. Enterocolitis, peritonitis. | Almost complete elimination. | Minimum. |
| **b** | Death 4 to 8 days post-injury. Enterocolitis, peritonitis. | Partial reduction. | Minimum. |
| **c** | Death 5 to 8 days post-injury. Enterocolitis, peritonitis. | Partial reduction. | Minimum. |
| **d** | Death 5 to 8 days post-injury. Enterocolitis, peritonitis. | Inconsistent. | Not detected. |
| **e** | 50% mortality at 9 days post-injury. Peritonitis, mucositis. | Inconsistent. | Not detected. |

As indicated in Table 2, regimen "a" was very effective, notably reducing and even completely eliminating fibrotic connective tissue, while at the same time generating abundant axonal branching that grew on astrocytes at the edge of the injury (Figure 4). The complete inhibition of fibrosis prevented the stumps of the spinal cord from merging, with a cavity measuring 1 to 1.5 mm in length which ruled out any possibility of axonal regeneration remaining. Nevertheless, tissue damage was minimal, except for the presence of isolated microhemorrhages.

Moreover, the effective regimen "a" was lethal in 100% of the cases between 6 and 9 days post-injury, and the animals likewise had enterocolitis, peritonitis, and mucositis. Most of the other combined regimens also caused a 100% mortality, and were furthermore much less effective. Only regimen "e" allowed half of the animals to survive beyond day 9 post-injury, but the antifibrotic effects were very inconsistent, with a slight reduction in spinal cord inflammation and fibrosis being shown in some cases and an increase being shown in other cases.

Overall, Examples 3 and 4 showed that it is not possible to obtain a safe and effective regimen for treating fibrosis caused by spinal cord injury by means of the systemic administration of SB743921 and imatinib. Furthermore, cavities remained in the injury site with the most effective regimens, indicating that it will be necessary to provide a substrate and stimulus for neural growth in any case.

### Example 5: Ineffectiveness of electrostimulation alone for repairing spinal cord in rats.

To find out the effect of electrostimulation alone on tissue repair, stimulation protocol was applied in the absence of other pharmacological treatments or implants at the injury site. Spinal cord transection was performed, creating the 2 mm gap as described in the preceding examples, and an electrode was implanted to administer electrical pulses to the injury site. The electrode consisted of a stainless steel microwire (50.8 µm in diameter) with Teflon insulation (A-M Systems) removed from the tip, leaving 1 mm of metal exposed. The metal tip was introduced in the subarachnoid space and fixed dorsally on the rostral edge of the injury. The dura mater was then sutured. Another electrode made of the same material, but with a larger metallic area without electrical insulation, was placed in the subcutaneous tissue of the animal's abdomen and was used as a counter electrode. The ends of the wires had gold pins that emerged from the dorsal skin of the animal for connection to the electrical pulse generator (Biologic VSP potentiostat/galvanostat).

Stimulation started 4 days post-injury and lasted for 10 days such that the animals were perfused at 14 DPI to extirpate the spinal cord and perform histological analysis like in the preceding examples. An electrical pulse protocol which induces arborization of healthy axons of the corticospinal tract when the cerebral cortex is stimulated was used (Brus-Ramer M, Carmel JB, Chakrabarty S, Martin JH. Electrical stimulation of spared corticospinal axons augments connections with ipsilateral spinal motor circuits after injury. J Neurosci. 2007;27:13793-801), but stimulating one and a half hours daily instead of 6 hours to prevent local reactions that may increase tissue damage. In summary, biphasic pulses of 200 µs/phase were applied, starting with the cathodic phase, in bursts of 45 ms at 333 Hz, repeated every 2 s, for one and a half hours daily for 10 days. Current intensity was maintained at the threshold which induced visible contraction of the paraspinal or thoracic muscles and was well tolerated without signs of pain in the animals, generally being 350 µA to 650 µA. The application of stimuli 150 µA above the motor threshold usually caused discomfort and stress to the animals, therefore the current was always kept within this limit, with a maximum of 750 µA. For biphasic pulses of 500 µA, the ohmic drop between electrodes was around -4/+2 V on the first day and - 7/+4 V after 10 days.

The stimulation protocol was well tolerated and no systemic adverse events were detected. The typical state of the rat spinal cord after 10 days of electrostimulation is shown in Figure 5. As can be observed, this treatment alone was insufficient to facilitate spinal cord repair. A fibrotic scar of about 1 mm thick sealed the stumps, preventing the growth of ascending and descending axonal tracts and astroglial migration across the injury site. Furthermore, cavities were formed in the spinal cord which were not observed in animals that did not receive any treatment (compare Figure 5 to Figure 1B).

### Example 6: Implantation of a fibrin hydrogel with SB743921 and electroconducting microfibers, and adjacent electrodes for injury site stimulation, in rats with spinal cord transection.

In Examples 3 to 5 it was concluded that systemic treatments with SB743921 and/or imatinib, or electrostimulation, performed in an isolated manner, did not provide a safe and effective treatment to reduce fibrosis and neural tissue gap after spinal cord transection in rats. Moreover, previous studies with implants having electroconducting microfibers embedded in an alginate or fibrin hydrogel, without the administration of drugs or electrostimulation, have shown that the microfibers aid in guided axonal growth across the spinal cavities, but the implant thereof is not effective in inducing supraspinal axon regeneration and also considerably increases tissue damage and stump separation, such that functional neural repair is not achieved (Alves-Sampaio A, Garcia-Rama C, Collazos-Castro JE. Biofunctionalized PEDOT-coated microfibers for the treatment of spinal cord injury. Biomaterials 2016; 89:98-113; Alves-Sampaio A, Del-Cerro P, Collazos-Castro JE. Composite Fibrin/Carbon Microfiber Implants for Bridging Spinal Cord Injury: A Translational Approach in Pigs. Int J Mol Sci. 2023;24:11102).

In order to prevent the systemic toxicity of the antifibrotic compounds, while at the same time providing a substrate for neural growth, a fibrin hydrogel with SB743921 and electroconducting microfibers was prepared *in vitro* and introduced in the injury site, and a microwire was implanted adjacent to the hydrogel (Figure 6A) to apply electrostimulation as described in Example 5. The dura mater and muscles were sutured and the wire terminals with gold pins were taken out through the dorsal skin of the animal (Figure 6A). The electrodes were connected daily to the potentiostat/galvanostat to apply electrical pulses (Figure 6B).

Conducting polymer-coated carbon microfibers were prepared and biofunctionalized with cell adhesion molecules using the protocols described in detail in previous patents and publications (P201231969, PCT/ES2013/070879; Collazos-Castro JE, García-Rama C, Alves-Sampaio A. Glial progenitor cell migration promotes CNS axon growth on functionalized electroconducting microfibers. Acta Biomater. 2016;35:42-56). In summary, carbon microfibers that are 7-µm in diameter, on which poly(3,4-ethylenedioxythiophene) (PEDOT) doped with poly[(4-styrenesulfonic acid)-*co*-(maleic acid)] (PSS-*co*-MA) was electrosynthesized (1 µA/mm² for 32 minutes), resulting in a PEDOT:PSS-*co*-MA coating, were used. The carboxylic groups of PSS-*co*-MA were reacted with EDC/NHS, and then with the amino groups of poly-L-lysine (PLL). Heparin, basic fibroblast growth factor (bFGF), and fibronectin, were added to the PLL layer.

For the preparation of the fibrin hydrogel, a solution containing human fibrinogen (Sigma F4883) and factor XIII (cluvot, CSL Behring 78779) was mixed in a 2:1 ratio with a solution containing thrombin (Sigma-Aldrich T7009) and CaCl₂ (2.6 mM, Sigma-Aldrich) in water with 0.9% (w/v) NaCl. The hydrogel with SB743921 and microfibers was prepared *in vitro* in tubes having a diameter of 2 mm, adding the microfibers in layers. The final concentrations of fibrinogen, thrombin, and factor XIII were 80 mg/ml, 33.3 U/ml, and 33.3 U/ml, respectively. 8 µl of a solution with SB743921 were added to the hydrogel at a final concentration of 0.6 mM.

### Example 7: Reduction of cavitation, facilitation of neural growth, and closure of tissue gap in rats with spinal transection and an implant consisting of fibrin/SB743921/microfibers combined with electrostimulation.

Twenty rats received transection and implantation of the fibrin hydrogel with SB743921, PEDOT:PSS-*co*-MA-coated biofunctionalized carbon microfibers, and electrodes adjacent to the hydrogel like in Example 6, and electrical stimulation was applied on half of the rats in order to find out whether the combined treatment exhibits any synergy. The animals were sacrificed 14 days post-injury to extirpate the spinal cord and perform histological analyses following the procedures described above.

The filler hydrogel of fibrin with SB743921 allowed good local control of fibrosis with excellent survival of the animals, but in the absence of electrical stimulation no reparative response was obtained. The fibrin degraded and the released drug inhibited fibrosis, but since there was no stimulus for neural growth, it produced an empty cavity with an extension very similar to the original (about 2 mm, Figure 7, left). However, after 10 days of electrostimulation, the cavity disappeared and the neural gap was considerably reduced (Figure 7, right), with the growth of several cell types, including neural cells, being observed. Numerous neurofilament-positive axons emerged from both stumps of the spinal cord and penetrated the center of the injury. The astrocytes of both stumps also aligned and migrated into the tissue gap, with there being only a separation of about 600 µm (Figure 7, right). Nevertheless, PDGFR+ connective tissue cells could still be found in the injury site, although in a smaller number than in the absence of treatment, said cells being oriented more longitudinally in the spinal cord and accompanied by axons and blood vessels. Seemingly, this secondary fibrotic tissue still partially inhibited axonal regeneration, preventing serotonergic axons from reaching the caudal stump of the spinal cord, at least at the time in which the animals were sacrificed (14 DPI).

In summary, the results of this example demonstrated that the use of a drug (SB743921)-releasing filler hydrogel for the neural tissue, combined with electrostimulation, can reduce fibrosis while at the same time promotes tissue gap closure and neural growth, without causing systemic toxicity or other relevant adverse effects after spinal cord transection in adult rats.

### Example 8: Therapeutic synergy between the drug-releasing electroactive implant and the systemic administration of imatinib in rats with spinal cord transection.

Next, the safety and usefulness of administering a hydrogel consisting of fibrin/SB743921/microfiber hydrogel + electrostimulation (ES) in the injury site, along with subcutaneous imatinib, was evaluated. The same methodology described in Example 7 was used, sacrificing the animals at 14 DPL for histological study.

Imatinib was dissolved in water at 100 mg/ml and then in a 0.9% (w/v) saline solution and injected subcutaneously. After having performed test 4 to find out the toxicity of the drugs, only 10 rats were used in this case and all the rats received the implant combined with ES, and furthermore they were distributed into 2 groups with the following imatinib regimens:
a) 4 doses of 5 mg/kg, every 12 hours, starting 12 hours post-injury.
b) Regimen "a" and additionally 5 mg/kg everyday until the end of the electrostimulation protocol.

Regimen "a" was completely safe for the animals when used with the implant, with there being no associated death nor the onset of adverse effects. Furthermore, histologically, a reduced presence of inflammatory cells could be confirmed in the perilesional spinal segments, although no significant effects were detected in terms of neural gap closure or axonal growth and glial migration.

Moreover, regimen "b" practically eliminated PDGFR+ residual fibrotic tissue from the injury site, leading again to the separation of the stumps of the spinal cord in two thirds of the cases. However, in the remaining one-third of the animals, an additional tissue gap closure with astrocyte migration and axonal growth could be verified. At 14 DPI, the distance between the astrocyte front of the stumps was reduced to about 400 µm (Figure 8), and the gap was even completely eliminated at some points and both the migratory astrocytes and growing axons went through the injury completely and reached the caudal stump. Furthermore, the SB743921-releasing electroactive implant combined with subcutaneous imatinib exhibited clear synergy, significantly increasing axonal extension and branching (Figure 8).

### Example 9: Application of a fibrin hydrogel with SB743921 and carbon microfibers, with a set of electrodes adjacent to the hydrogel, for the treatment of neural damage caused by spinal cord contusion in pigs.

To successfully repair human neurological injuries, it is essential to evaluate the therapeutic strategies in large animals. The feasibility of the clinical application of the present invention was validated in two porcine models of spinal cord injury, such as contusion in the thoracolumbar junction (T14-T15), and the cervical hemisection (C6). Contusion reproduces neuronal loss and tissue cavitation patterns found in more than 60% of patients with spinal cord injury (Collazos-Castro JE, Soto VM, Gutiérrez-Dávila M, Nieto-Sampedro M. Motoneuron loss associated with chronic locomotion impairments after spinal cord contusion in the rat. J. Neurotrauma 2005; 22:544-558). The cervical spinal hemisection models the laceration damage of the neural tissue and furthermore the most common location of spinal injuries. When the hemisection is combined with neural tissue extirpation, the pathological process of tissue cavitation is also reproduced (Cerro PD, Barriga-Martín A, Vara H, Romero-Muñoz LM, Rodríguez-De-Lope Á, Collazos-Castro JE. Neuropathological and motor impairments after incomplete cervical spinal cord injury in pigs. J Neurotrauma. 2021; 38:2956-2977).

This example describes the methodology for causing spinal contusions in pigs, and the use of the present invention for the treatment of said injuries is illustrated. For this test, the results of histological and functional repair of which are shown in Example 10, 18 female pigs of the great white English pig breed (*Sus scrofa domesticus*) of 2 months of age and weighing 14 to 21 kilograms at the time of injury were used, distributed into three experimental groups:
1) spinal cord contusion without treatment (n = 6).
2) contusion, myelotomy at 24 hours post-injury, washing with a SB743921 solution and treatment with a hydrogel consisting of fibrin, SB743921, and microfibers, with subcutaneous imatinib, but without ES (n = 6).
3) the same treatment 2, with electrodes being positioned adjacent to the hydrogel and applying electrostimulation for 10 days (n = 6). The ES protocol was the same used for the rats in Examples 5, 6, and 7, but the daily duration was increased from 1.5 hours to 6 hours.

The animals were acquired from the company Granja Agropardal, specializing in raising pigs for research. The experimental procedures were approved by the Ethics Committee on Experimentation (CEEA), the Authorized Body of Toledo, and the Ministry of Agriculture and Environment of the JCCM. All surgical procedures were performed under inhalation anesthesia following the protocol described in Alves-Sampaio A, Del-Cerro P, Collazos-Castro JE. Composite Fibrin/Carbon Microfiber Implants for Bridging Spinal Cord Injury: A Translational Approach in Pigs. Int J Mol Sci. 2023;24:11102. Said publication also describes in detail the automated closed loop system used for porcine spinal cord contusion, and the general care for the animals.

The surface hitting the spinal cord was flat and circular, with diameter of 8 millimeters (Figure 9A). The peak force of the impact was set at 30 N. After contusion, the muscles were closely sutured in layers to prevent the formation of seromas, and finally the skin was closed. A urinary catheter was placed to evacuate and quantify the urine produced until the recovery of spontaneous micturition.

Twenty-four hours after contusion, the animals were anesthetized again, the injury site was exposed (Figure 9B), and a longitudinal incision was made in the dura mater and the pia. After incision, part of the devitalized tissue was spontaneously evacuated as it was under pressure from edema at the contusion site (Figure 9C). Next, the injured site was irrigated several times with saline solution using a fine cannula, removing most of the cell debris. Subsequently, the injury was washed with a SB743921 solution at a concentration of 5 µM, which was left to act for 10 minutes to impregnate the perilesional site. A fibrin hydrogel preformed with SB743921 and conducting polymer-coated carbon microfibers was then introduced (Figure 9D). The rest of the cavity was filled with the same type of hydrogel gelled *in vivo*, in this case reducing the final concentrations of fibrinogen, thrombin, and factor XIII (about 22.2 mg/ml, 11.1 U/ml, and 11.1 U/ml, respectively). Lastly, electrodes (stainless steel microwires) were positioned on the hydrogel (Figure 9E), and the dura mater was sutured (Figure 9F).

Surgical reintervention 24 hours post-injury was successful in all cases, with no deaths or postoperative complications associated with the procedure. In compliance with CEEA guidelines, all animals received treatment to reduce postoperative soft tissue and osteoarticular pain and inflammation with meperidine (4 mg/kg SC) every 12 hours for two days, and meloxicam (0.2 mg/kg SC) for 7 days. They were also administered marbofloxacin (2 mg/kg IM) as antibiotic for 7 days. Additionally, pigs in groups 2 and 3 received 9 doses of imatinib (1.4 mg/kg, SC), starting 6 hours after implantation, every 12 hours for the first 4 doses, and every 24 hours for the next 5 doses.

### Example 10: Neural repair improvement and motor function recovery in pigs with spinal cord contusion that received a fibrin hydrogel with SB743921 and carbon microfibers, combined with electrostimulation of cathodic start, through electrodes adjacent to the hydrogel.

The histological and functional effects of the procedures described in Example 9 are described. The animals received inhalation anesthesia to surgically expose the injured spinal segment and the adjacent segments rostrally and caudally. Subsequently, they were given a lethal dose of pentobarbital (120 mg/kg IV) and spinal segments T13 to L3 were extirpated. The tissue was fixed by immersion in 4% paraformaldehyde in 0.1 M PBS for 4 days. The spinal segments were dissected and immersed separately in PBS with 30% sucrose for 4 days and then embedded in OCT^{®} and stored at -20°C until processing. The tissue was cut in a cryostat into horizontal sections measuring 10 µm for immunohistochemical staining, and 50 µm for eriochrome cyanine and cresyl violet staining. Histological studies were performed for the purpose of evaluating neural damage, tissue cavitation, axonal and glial growth, and invasion of the injury by fibrotic connective tissue cells. For immunohistochemistry, the same procedures and antibodies described in Examples 1 and 3 were used.

The injury spanned about 1 cm longitudinally, and encompassed most of the spinal cord dorsoventrally, although it usually left a small portion of the white matter healthy in the ventrolateral region on one side. In control animals, necrotic tissue was reabsorbed leaving only some remnants of fragmented axons and a large central cavity surrounded by fibrosis (Figure 10A), with no neural regeneration across the cavity (Figures 10A1 and 10B). Furthermore, the spinal tissue on either side of the injury showed a considerable degree of atrophy (Figure 10A).

Treatment with hydrogel consisting of fibrin/SB743921/microfibers, combined with imatinib and electrostimulation, facilitated impressive neural growth in the damaged area (Figures 10C and 10D). Perilesional spinal segments hypertrophied and moved closer to one another, reducing the cavity (Figure 10C). Furthermore, a bundle with thousands of axons regenerated across the cavity, following the microfibers and connecting the two sides of the spinal cord (Figures 10 C1 and C2). In the most ventral area where no microfibers were implanted, neural regeneration was more limited, but in any case serotonergic and noradrenergic supraspinal axons branched copiously and invaded the residual cavity in a disorganized manner (Figure 10D).

Functional recovery of pigs was evaluated by means of the PTIBS scale described in Lee J.H., Jones CF, Okon EB, Anderson L, Tigchelaar S, Kooner P, Godbey T, Chua B, Gray G, Hildebrandt R, Cripton P, Tetzlaff W, Kwon BK. A novel porcine model of traumatic thoracic spinal cord injury. J Neurotrauma. 2013;30:142-59. On this scale, a score of 1 indicates hind leg paralysis, and 10 points denote normal locomotion. The functional recovery of the three treatment groups is shown in Figure 11. The implant combined with ES improved motor function in pigs with spinal cord contusion, with significant differences (p<0.05) at days 14, 21, and 28 post-injury with respect to the untreated control group. All animals receiving the combined treatment had improved 3 to 4 points at the end of the 10 days of electrostimulation, while the controls improved 1 to 2 points.

### Example 11: Neural repair improvement in pigs with spinal cord contusion that received a fibrin hydrogel with SB743921 and carbon microfibers, combined with biphasic electrical pulses of anodic start.

In Example 10, the biphasic electrical pulses were always applied starting from the negative (cathodic) phase. An additional group of pigs was used to find out whether the neuro-regenerative effect is obtained exclusively with said electrical polarity, or whether it can also be achieved with biphasic electrical pulses starting from the positive (anodic) phase. The other experimental conditions were exactly the same as those described in Examples 9 and 10. As illustrated in Figure 12, ES starting from the anodic phase also induced hypertrophy of the perilesional neural tissue and abundant axonal regeneration across the spinal cord cavity, connecting both sides of the spinal cord. No adverse effects that can be attributed to the treatment were recorded.

### Example 12: Antifibrotic and neural growth-promoting effect of the hydrogel implant consisting of fibrin/SB743921 with another support material in a cervical spinal hemisection with neural tissue excision in pigs.

In severely damaged sites of the nervous system, for example, in large continuity solutions of neural tissue due to massive laceration, abrasion, or compression, it may be necessary to use additional support materials for the drug-releasing hydrogel for the purpose of reinforcing its mechanical strength or providing additional guidance for tissue growth, or sites to anchor the implant to host structures.

This example demonstrates that the hydrogel consisting of fibrin/SB743921 formed *in vitro* inside a second material, in this case a silicone tube, and implanted therewith, is useful for limiting fibrosis and secondary tissue damage, and for initiating axonal growth responses following an open injury on the porcine spinal cord, similar to a laceration with loss of neural tissue.

A recently described model of porcine cervical spinal hemisection with the extirpation of 1 cm of neural tissue from the C6 cervical spinal segment was used (Cerro PD, Barriga-Martín A, Vara H, Romero-Muñoz LM, Rodríguez-De-Lope Á, Collazos-Castro JE. Neuropathological and motor impairments after incomplete cervical spinal cord injury in pigs. J Neurotrauma. 2021; 38:2956-2977).

For this test, 6 pigs distributed into two experimental groups were used:
1) Spinal cord injury and implantation of a silicone tube with fibrin hydrogel without drug (n = 3).
2) Spinal cord injury and implantation of a silicone tube with fibrin hydrogel formed with SB743921 (n = 3).

Fibrin with SB743921 was gelled *in vitro* inside a tube with a morphology similar to the hemi-medulla of the C6 segment, the wall of which was a flexible medical-grade silicone membrane of about 100 µm thick. The concentrations of the compounds described in Examples 6 and 9 were used for the hydrogel and drug.

The implant was introduced immediately after creating the cavity in the spinal cord, and the dura mater was then sutured to keep it in place. One month after the injury, the animals received inhalation anesthesia to surgically expose the cervical cord. Subsequently, they were given a lethal dose of pentobarbital (120 mg/kg IV) and spinal segments C1 to T2 were extirpated. Tissue was fixed and processed following the methodology described in Example 10. Neural damage, tissue cavitation, axonal and glial growth, and invasion of the injury by fibrotic connective tissue cells, were evaluated. For immunohistochemistry, the same procedures and antibodies described in Examples 1, 3, and 10 were used.

As illustrated in Figure 13, the implant was kept in place and the hydrogel inside the silicone tube was only partially degraded in both groups. The implant that did not contain the drug caused tissue damage and significant fibrosis impenetrable to regenerating axons. Bundles of dense fibrotic tissue 1 to 2 mm thick, originating mainly in the meninges, formed transversely in the stumps of the spinal cord and also extended around and into the silicone tube, blocking any possibility of neural growth. In some cases, contraction of the fibrotic scar apparently widened the silicone tube, causing neural tissue compression and additional damage.

The tissue response was much better in animals that received the SB743921-releasing implant. In this case, fibrosis was very significantly reduced, being eliminated at some points, and showing a residual thickness of about 20% of that observed in the animals with fibrin but without drug. Furthermore, axons reached the edge of the tissue and began to grow in the most peripheral portion of the hydrogel (Figure 13). The reduced fibrotic scar did not contract, such that there was also no compression and tissue damage due to deformation of the silicone tube.

### Example 13: Implantation of a fibrin hydrogel with SB743921 and an assembly of interconnected electroconducting microfibers, combined with epidural electrodes in a spinal hemisection with neural tissue excision in pigs.

Lastly, 4 pigs received cervical spinal hemisection and excision of 1 cm of neural tissue using the methodology described in Example 12, for the purpose of evaluating the feasibility of implanting the hydrogel consisting of fibrin/SB743921 and containing an assembly of interconnected microfibers for electrical stimulation, aligned longitudinally in the spinal cord. In addition, epidural electrodes were sutured to the dura mater adjacent to the injury site to complete the electrostimulation circuit and to measure the resulting electrical parameters.

Using the methodology described in Example 6, the conducting polymer PEDOT:PSS-*co*-MA was deposited and functionalized on an assembly of interconnected carbon microfibers through a metallic microwire (AXON' Cable SAS, France) having a diameter of 12.8 µM (Figure 14A). The microwire was insulated with silicone once the carbon microfibers were bonded. The microfibers were trimmed to a final length of 12 mm prior to polymer electrodeposition. The electrical functionality of the assembly was evaluated before and after electrodeposition, as well as after inclusion in the fibrin hydrogel, confirming the increased charge transfer due to the polymer sheath of the microfibers. Before introducing the implant in the tissue defect, the site was washed with a SB743921 solution at a concentration of 5 µM, which was left to act for 10 minutes. These pigs were also administered 4 subcutaneous doses of imatinib (1.4 mg/kg), every 12 hours starting 8-12 hours after injury.

The preformed hydrogel consisting of fibrin/SB743921/assembly of microfibers was successfully implanted into the tissue defect (Figure 14B), without losing electrical interconnections. Once implantation was performed and the durorrhaphy completed, stainless steel electrodes (discs with a diameter of 3 mm) were sutured on the dura mater about 5 mm rostrally and caudally with respect to the edges of the injury (Figure 14C). No adverse effects due to the implant were observed. Complete inhibition of fibrosis around the assembly of interconnected microfibers, as well as partial closure of the tissue gap one month after injury, were verified.

## Claims

1. A drug-releasing electroactive volumetric implant comprising:
a) a hydrogel made of natural or synthetic polymers selected from the list comprising: polypeptides, fibrin, fibronectin, collagen, proteoglycans, glycosaminoglycans, alginate, chitosan, acrylamide, methacrylate, hydrogelatin, derivatives and combinations of these materials;
b) at least one drug incorporated in the preceding hydrogel, wherein one of the drugs is a compound which blocks or inhibits the function of kinesins, or reduces the expression thereof;
c) a set of electrodes for applying electrical stimuli on injured tissue, wherein at least one electrode is positioned inside the hydrogel or adjacent thereto.

2. The implant according to claim 1, wherein the compound which blocks or inhibits the function of kinesins, or reduces the expression thereof, is a kinesin Eg5 inhibitor.

3. The implant according to claim 2, wherein the kinesin Eg5 inhibitor is the drug SB743921.

4. The implant according to any of preceding claims 1 to 3, wherein the set of electrodes (c) is arranged inside the hydrogel.

5. The implant according to any of preceding claims 1 to 4, wherein the electrodes positioned inside the hydrogel comprise or consist of: carbon microfibers, carbon nanostructures, or microwires made of metals or metal oxides, composites, or metal alloys.

6. The implant according to any of preceding claims 1 to 5, wherein the electrodes are coated with conducting polymers.

7. The implant according to claim 6, wherein the conducting polymer coating the electrodes is selected from poly(3,4-ethylenedioxythiophene) (PEDOT), polypyrrole, or polyaniline.

8. The implant according to any of preceding claims 1 to 7, wherein the surface of the electrodes is functionalized with at least one molecule with biological activity to help in neural growth responses on or around the electrode.

9. The implant according to claim 6 or 7, wherein the conducting polymer is functionalized with at least one molecule with biological activity to help in neural growth responses on or around the electrode.

10. The implant according to any of claims 8 or 9, wherein the molecule with biological activity used for the functionalization of the electrodes or of the conducting polymer is selected from the list comprising: cell adhesion molecules, molecules of the extracellular matrix, growth factors, netrins, ephrins, semaphorins, protocadherins or Slit or Wnt proteins, morphogens, proteoglycans, glycosaminoglycans, gangliosides, proteins, peptides, or combinations thereof.

11. A method for preparing an implant defined according to any of preceding claims 1 to 10, which comprises preparing a fibrin hydrogel by means of mixing:
- a first aqueous solution containing human fibrinogen at concentrations of between 0.5 and 100 mg/ml and factor XIII between 3 and 300 U/ml; and
- a second aqueous solution containing thrombin at concentrations of between 3 and 300 U/ml and CaCl₂ at concentrations of between 0.26 and 26 mM,
wherein at least one of these solutions contains a drug which blocks or inhibits the function of kinesins, or reduces the expression thereof.

12. The method according to preceding claim 11, wherein the mixing of the precursor solutions of the hydrogel is performed *in vitro*.

13. The method according to any of claims 11 to 12, wherein at least one of the precursor solutions of the hydrogel contains the set of electrodes or at least one electrode.

14. The method according to preceding claim 13, wherein the drug is first encapsulated in particles, vesicles, liposomes, or the like for the purpose of increasing its retention or improving its activity, and then incorporated in the precursor solutions of the hydrogel.

15. The method according to any of claims 11 to 14, wherein electrodes selected from microfibers, microwires, or other electroconducting elements are additionally incorporated aligned in the hydrogel, during the gelling process.

16. The implant as described in any of claims 1 to 10 for use thereof in repairing neural tissue or the nervous system.

17. The implant for use thereof according to claim 16, by means of a surgical procedure comprising the following steps:
a) cleaning the neurological injury site of cell debris, blood, and fibrotic adhesions;
b) implanting the hydrogel in the neurological injury site cleaned according to step a) during gelling when it occurs *in vivo*, or after gelling when it occurs *in vitro*;
c) positioning at least one electrode adjacent to the hydrogel, when no electrodes have been incorporated inside the hydrogel implanted in step b, or when no electrodes have been implanted in the neurological injury site prior to step b;
d) supplying electrical stimuli through the electrodes.

18. The implant for use thereof according to preceding claim 17, wherein in addition to step a) the injury site is washed and impregnated with a solution containing the same pharmacological compound which blocks or inhibits the function of kinesins, or reduces the expression thereof; present in the hydrogel.

19. The implant for use thereof according to any of claims 17 to 18, wherein the hydrogel is formed with the pharmacological compound *in vitro* and is then implanted in the neurological injury site cleaned according to step a).

20. The implant for use thereof according to preceding claim 19, wherein the hydrogel with the pharmacological compound is prepared inside a second implantable material, and the resulting composite implant is introduced in the damaged region of the nervous system cleaned according to step a).

21. The implant for use thereof according to any of claims 17 to 18, wherein the hydrogel is formed with the pharmacological compound *in vivo*, such that gelling takes place directly in the neurological injury site cleaned according to step a).

22. The implant for use thereof according to any of claims 17 to 18, wherein the hydrogel is prepared *in vitro* with the pharmacological compound, and electrodes are additionally introduced therein during gelling, and is then implanted in the neurological injury site according to step b).

23. The implant for use thereof according to any of claims 17 to 18, wherein the electrodes are implanted in the neurological damage site conditioned in step a), and the hydrogel with the pharmacological compound is then formed in vivo according to step b), surrounding the implanted electrodes.

24. The implant for use thereof according to any of claims 17 to 18, wherein the hydrogel with the pharmacological compound is formed *in vivo* inside the tissue defect according to step b), and electrodes are positioned adjacent to said hydrogel, inside the nervous system or on the membranes enveloping the nerve tissue.

25. The implant for use thereof according to any of claims 16 to 24, wherein biphasic electrical stimuli or monophasic electrical stimuli are applied through the electrodes of the implant.

26. The implant for use thereof according to any of claims 16 to 25, wherein electrical stimuli are applied through the electrodes which start or consist of an electric current having a positive polarity or a negative polarity.

27. The implant for use thereof according to any of claims 16 to 26, wherein electrical stimuli are applied through the electrodes in which the intensity of the electric current applied, in absolute value, is comprised between 1 nanoampere and 20 milliamperes.

28. The implant for use thereof according to any of claims 16 to 27, wherein electrical stimuli are applied through the electrodes in which the frequency is comprised between 0.00001 Hz and 100000 Hz.

29. The implant for use thereof according to any of claims 16 to 28, wherein said implant is used in combination with additional pharmacological compounds administered locally or systemically to promote implant integration and neural repair.

30. The implant for use thereof according to preceding claim 29, wherein the pharmacological compound is selected from an anti-inflammatory drug, antifibrotic, antibiotic, neuroprotector, and a neural growth promoter.

31. The implant for use thereof according to preceding claim 30, wherein the pharmacological compound is a tyrosine kinase receptor inhibitor selected from imatinib, sunitinib, sorafenib, masitinib, dasatinib, nilotinib, gefitinib, erlotinib, lapatinib, and tofacitinib.
